# EUROPEAN PATENT SPECIFICATION

(11) **EP 0 425 212 B1**
(45) Date of publication and mention of the grant of the patent: **07.04.1999**
(21) Application number: 90311537.6
(22) Date of filing: 22.10.1990
(51) Int. Cl.: C07K 5/12, C07K 5/08, C07K 5/10, C07K 7/06, C07K 14/75, A61K 38/12

(54) **Cyclic anti-aggregatory peptides**
Zyklische Peptide mit aggregationshemmenden Eigenschaften
Peptides cycliques à activité anti-aggrégante

(30) Priority: 23.10.1989 US 425906
(43) Date of publication of application: 02.05.1991
(73) Proprietor: SMITHKLINE BEECHAM CORPORATION, Philadelphia Pennsylvania 19101 (US)
(72) Inventor: Ali, Fadia El-Fehail, King of Prussia PA19406 (US); Samanen, James Martin, Collegeville PA 19426-0989 (US)
(74) Representative: Waters, David Martin, Dr.

(56) References cited:
- EP-A- 0 275 748
- EP-A- 0 341 915
- EP-A- 0 410 540
- EP-A- 0 410 541
- WO-A-89/05150
- WO-A-90/15620
- WO-A-91/11548
- WO-A-91/15224
- WO-A-92/00995

## Description

### Field of the Invention

This invention relates to novel peptides which inhibit platelet aggregation, pharmaceutical compositions containing the peptides and methods of using the peptides. In particular, a method of using the peptides of this invention in combination with fibrinolytic agents is disclosed.

### Background of the Invention

A thrombus is the result of processes which initiate the coagulation cascade. It is composed of an aggregation of platelets enmeshed in a polymeric network of fibrin. This process is normally initiated as a consequence of tissue injury and has the effect of slowing or preventing blood flow in a vessel. Etiological factors which are not directly related to tissue injury, such as atherosclerotic plaque, inflammation of the blood vessels (phlebitis) and septicemia, may also initiate thrombus formation. In some instances, the inappropriate formation of a thrombus, and subsequent decrease in blood flow, may have pathological consequences, such as stroke, pulmonary embolism and heart disease.

Platelets play a major role in thrombus formation. Current antithrombotic therapy employs agents that modify the platelet/endothelial cell arachidonate-prostaglandin system, such as prostacyclin analogues, cyclooxygenase inhibitors, thromboxane synthesis inhibitors and thromboxane receptor antagonists; and anti-coagulants, such as heparin. These agents inhibit one or both of two discernible phases of platelet aggregation. The primary phase, which is a response to chemical stimuli, such as ADP (adenosine diphosphate), collagen, epinephrine or thrombin, causes initial activation of the platelets. This is followed by a secondary phase, which is initiated by the platelets themselves, and is characterized by thromboxane A₂ (TxA₂) synthesis and the release of additional ADP from platelet storage granules, which further activates platelets.

Prostacyclin, also called prostaglandin I₂ (PGI₂), and stable PGI₂ analogues inhibit both the primary and secondary phases of platelet aggregation However, use of such analogues has been associated with undesirable changes in blood pressure. See Aiken, et al., Prostaglandins, 19, 629-43 (1980).

Cyclooxygenase inhibitors and thromboxane synthetase inhibitors act to block the production of TxA₂. TxA₂ antagonists block the effects of TxA₂ by binding the TxA₂ receptor. These therapies act only upon the secondary stage of platelet activation. Use of cyclooxygenase inhibitors has been associated with ulcerogenesis and an adverse effect upon prostacyclin synthesis.

Heparin prevents the activation of fibrinogen by thrombin and thereby prevents the activation of the GPIIb-IIIa receptor by thrombin. This inhibits only the primary phase of platelet aggregation and has little effect upon activation of platelets by other means, such as collagen, ADP and epinephrine.

Cyclooxygenase inhibitors, prostaglandin analogues and heparin all inhibit platelet aggregation indirectly by inhibiting the primary or secondary phase of platelet/fibrinogen activation. There is therefore a need for selective therapeutic products which block platelet aggregation directly, whether it arises from the primary or secondary phase of platelet activation.

Platelet aggregation is believed to be mediated primarily through the GPIIb-IIIa platelet receptor complex. Von Willebrand factor, a plasma protein, and fibrinogen are able to bind and crosslink GPIIb-IIIa receptors on adjacent platelets and thereby effect aggregation of platelets. Fibronectin, vitronectin and thrombospondin are proteins which have also been demonstrated to bind to GPIIb-IIIa. Fibronectin is found in plasma and as a structural protein in the intracellular matrix. Binding between the structural proteins and GPIIb-IIIa may function to cause platelets to adhere to damaged vessel walls.

The importance of the GPIIb-IIIa receptor to platelet aggregation has been demonstrated by methods which mask the receptor. Thus, Coller et al. (Blood, 66, 1456-9 (1985)) have shown that antibodies to this complex inhibit platelet aggregation in dogs induced by ADP.

Peptide fragments of human plasma fibronectin and synthetic peptides containing the RGD sequence which promote cell attachment and enhance phagocytosis are disclosed in U.S. Patent 517,686, U.S. Patent 4,589,881, U.S. Patent 4,661,111 and U.S. Patent 4,614,517. Linear and cyclic peptides containing an RGD sequence have also be reported in WO 89/05150 (PCT US88/04403). Peptides which contain an RGD sequence have been reported to inhibit platelet aggregation. Nievelstein et al. (Thromb. and Hemostasis, 58, 2133(1987)) have reported that -RGDS- peptides inhibit thrombin induced aggregation and adhesion of platelets to fibronectin, and may interact through the GPIIb-IIIa complex. U.S. Patent 4,683,291 discloses peptides containing Arg and Lys and an -RGD- sequence which inhibit binding of fibrinogen to platelets and inhibit platelet aggregation. A disadvantage of these peptides is their poor stability in plasma and their low potency.

WO 89/05150 (PCT/US88/04403; La Jolla Cancer Research Foundation) discloses certain heterodetic (disulfide and amide) peptides containing the sequence Arg-Gly-Asp, which inhibit the binding of vitronectin to normal rat kidney cells.

EP-A 275748 (INSERM) discloses certain heterodetic (disulfide) peptides of the formula cyclo (S,S) Cys-X²-Gly-Asp-X³-Cys which inhibit platelet aggregation. EP-A 410541 (Merck) filed 25.07.90 discloses certain heterodetic disulfide peptides and homodetic peptides containing an 7-aminoheptanoic acid residue, which inhibit platelet aggregation. EP-A 410540 (Merck) filed 25.07.90 discloses certain heterodetic (disulfide) peptides, and homodetic peptides containing gamma-amino butyric acid or 7-aminohexanoic acid residue, which contain an Arg-Gly-Asp sequence. WO 90/15620 (COR Therapeutics) filed 15.06.90 discloses homodetic and heterodetic (disulfide) peptides which contain the sequence K*-(Gly/Sar)-Asp, wherein K* is a lysine or modified lysine residue. EP-A 406428 (Asahi) filed 08.09.89, published 22.03.90, discloses homodetic peptides containing the sequence Arg-Gly-Asp, which inhibit the binding of human epithelial cancer cells to vitronectin and fibronectin, and platelet aggregation. WO 91/15224 (SmithKline Beecham) priority date 30.03.90, discloses certain homodetic and heterodetic (disulfide) peptides containing the Arg-Gly-Asp sequence which inhibit the binding of the HIV tat protein to L8 (myoblastic), HUT-78 (T-lymphocytic), MOLT-4 (T-lymphocytic) and THP-1(myelomonocytic) cells. WO 92/00995 (Tanabe Seiyaku) priority date 09.07.90, discloses homodetic and heterodetic (disulfide, thioether, amide) peptides which inhibit leukocyte adhesion to endothelial cells.

Other linear and cyclic peptides, the disclosure of which are incorporated herein by reference, are reported in EP-A 0 341 915 (SmithKline Beckman Corporation) filed 05.05.89. These cyclic structures comprise a disulfide ring formed by two sulfhydryl-bearing aliphatic amino acid residues.

The instant invention provides cyclic compounds in which the cyclic structure comprises a homodetic peptide wherein the ring is formed by a peptide bond, or a heterodetic peptide wherein the ring is formed by an alkylene, sulfide or disulfide bridge. This invention further discloses cyclic compounds in which the unit B-Gly-Asp-Q, as hereinafter defined in formula (I), is repeated more than once. Construction of such unusual ring structures surprisingly results in pharmacologically active compounds. It has further been discovered that neither a terminal amino group or a terminal carbonyl group is required for anti-aggregatory activity. The compounds of this invention are further resistant to plasma proteases and show a selectivity for inhibition of binding to the fibrinogen receptor over other integrin receptors; such as vitronectin or fibronectin. Thus, an advantage to the compounds of this invention is their ability to inhibit platelet aggregation without appreciably inhibiting the adhesion of platelets to other integrin receptors.

Recent advances for treatment of occluded arteries and deep vein thrombosis employ fibrinolytic agents to lyse thrombi or emboli in order to reestablish or improve blood flow. Fibrinolytic agents, such as tissue plasminogen activator (tPA), urokinase (UK), pro-Urokinase(pUK), and streptokinase (SK), and mutants and derivatives thereof, are proteolytic enzymes which cause fibrin to be hydrolyzed at specific sites and thereby fragment the fibrin network. Their action in vivo is to proteolytically activate plasminogen in the blood to form plasmin, which causes lysis of the fibrin clot. Lysis of fibrin into smaller peptides has the effect of solubilizing the thrombus or embolus. A recurrent problem with such therapy, however, is the reocclusion of the blood vessel due to formation of a secondary thrombus.

Fibrinolytic therapy is most commonly used for reestablishing flow in a thrombosed blood vessel. However, fibrinolytic therapy does not reverse the factors responsible for the initiation of the thrombus. For this reason, anticoagulants such as heparin are often used to prevent reocclusion. In fact, patients which have a high degree of stenosis in an artery are at extremely high risk of rethrombosis after reperfusion, even in the presence of high doses of heparin. See Gold et al., Circ., 73, 347-52 (1986). In addition, use of SK and tPA has been associated with platelet hyperaggregability. See Ohlstein, et al., Thromb. Res., 4, 575-85 (1987). Treatment with higher doses of tPA can be associated with systemic bleeding and is not recommended for preventing reocclusion. There is, therefore, a need for a method for preventing rethrombosis after fibrinolytic therapy.

U.S. Patent Application Serial No. 917,122 discloses TxA2 antagonists for use in a method for inhibiting reocclusion following reperfusion and for lowering the dose of tPA required for fibrinolysis. Yasuda et al. (Clin. Res., 34, 2 (1986)) have demonstrated that reocclusion by fibrin rich platelet thrombi, after thrombolysis with tPA, may be inhibited by a murine monoclonal antibody to GPIIb-IIIa in dogs. This invention discloses a new method for inhibiting reocclusion of a blood vessel by administering peptides which directly inhibit platelet aggregation.

### Summary of the Invention

In one aspect this invention is a compound of the formula (I): 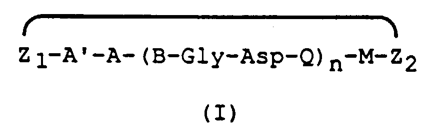
Z1-A'-A-(B-Gly-Asp-Q)n-M-Z2

(I)
 wherein:
A' is absent, Asn, Gln, Ala or Abu;
A is absent or a D- or L- amino acid chosen from Arg, HArg, NArg, (Me₂)Arg, (Et₂)Arg, Abu, Ala, Gly, His, Lys, or an α-R' substituted derivative thereof, Dtc, Tpr or Pro;
B is a D- or L- amino acid chosen from Arg, HArg, (Me₂)Arg, (Et₂)Arg, Lys or an α-R' substituted derivative thereof;
Q is absent or a D or L amino acid chosen from Tyr, (Alk)Tyr, Phe, (4'W)Phe, HPhe, Phg, Pro, Trp, His, Ser, (Alk)Ser, Thr, (Alk)Thr, (Alk)Cys, (Alk)Pen, Ala, Val, Nva, Met, Leu, Ile, Nle or Nal, or an α-R' substituted derivative thereof;
M absent or Gly or D or L Glu, Phe, Pro, Lys, Ser or, provided n is 1, B-Gly-Glu-Q;
W is halogen or Alk;
R' is Alk or PhCH₂;
Z₁ is 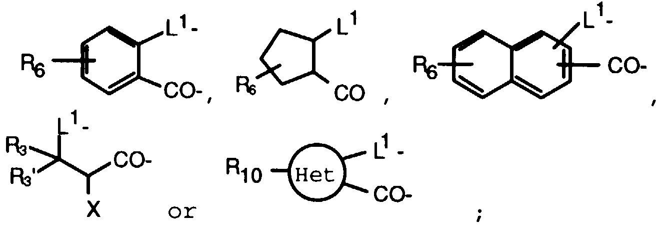
Z₂ is 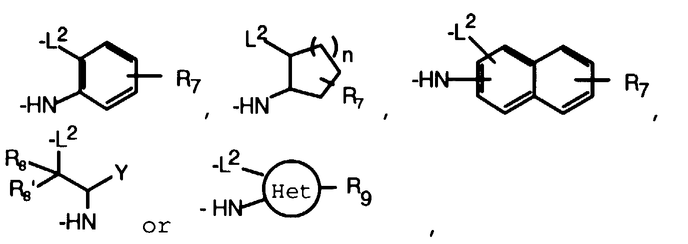 wherein Z₁ and Z₂ are linked via a covalent bond between L¹ and L²;
L¹ and L² are -S-;
X is R₄R₅N;
Y is H, CONR₁R₂ or CO₂R₂;
R₁ and R₂ are H, Alk or (CH₂)ₚAr;
R₃ and R_{3'} are H, Alk, (CH₂)ₚAr or taken together are -(CH₂)₄- or -(CH₂)₅-;
R₄ is H or Alk;
R₅ is R₁₁, R₁₁CO, R₁₁OCO, R₁₁OCH(R_{11'})CO, R₁₁NHCH(R_{11'})CO, R₁₁SCH(R_{11'})CO, R₁₁SO₂ or R₁₁SO;
R₆ is Alk, OAlk, halogen or X;
R₇ is H, Alk, OAlk, halogen or Y;
R₈ and R_{8'} are H, Alk, (CH₂)ₚPh, (CH₂)ₚNph or taken together are -(CH₂)₄- or -(CH₂)₅-;
R₉ is H, Alk or Y;
R₁₀ is H or Alk;
R₁₁ and R_{11'} are H, C₁₋₅alkyl, C₃₋₇cycloalkyl, Ar, Ar-C₁₋₅alkyl, Ar-C₃₋₇cycloalkyl;
Ar is phenyl or phenyl substituted by one or two C₁₋₅alkyl, trifluoromethyl, hydroxy, C₁₋₅alkoxy or halogen groups;
Alk is C₁₋₄alkyl;
Het is a heterocycle selected from pyridine, pyrrole, pyrrolidine, imidazole, triazole, thiophene, furan and thiazole, wherein L¹ is ortho to -CO- and L² is ortho to -NH-;
n is 1 or 2; and
p is 0, 1, 2 or 3
and pharmaceutically active salts thereof;
provided that when Z₁ is -L¹-C(R₃)(R_{3'})-CH(X)-CO-, R₃ and R_{3'} are H or Alk or together are -(CH₂)₄- or -(CH₂)₅- and Z₂ is -L²-C(R₈)(R_{8'})-CH(Y)-NH-, R₈ and R_{8'} are not both H or Alk nor together are -(CH₂)₄- or -(CH₂)₅-; and provided that M is absent only when Q is absent and only when Z₂ is

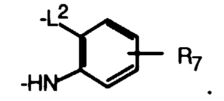

In another aspect this invention is a compound of formula (IA): 
Z1-A'-A-(B-Gly-Asp-Q)nM-Z2
(IA)
 wherein:
A' is absent, Asn, Gln, Ala or Abu;
A is absent or a D- or L- amino acid chosen from Arg, HArg, NArg, (Me₂)Arg, (Et₂)Arg, Abu, Ala, Gly, His, Lys, or an α-R' substituted derivative thereof, Dtc, Tpr or Pro;
B is a MeArg;
Q is absent or a D or L amino acid chosen from Tyr, (Alk)Tyr, Phe, (4'W)Phe, HPhe, Phg, Pro, Trp, His, Ser, (Alk)Ser, Thr, (Alk)Thr, (Alk)Cys, (Alk)Pen, Ala, Val, Nva, Met, Leu, Ile, Nle or Nal, or an α-R' substituted derivative thereof;
M is Gly or D or L Glu, Phe, Pro, Lys, Ser or, provided n is 1, B-Gly-Glu-Q;
W is halogen or Alk;
R' is Alk or PhCH₂;
n is 1 or 2;
Alk is C₁₋₄alkyl;
Z₁ and Z₂ are, taken together, a covalent bond between the amino terminal residue and the carboxy terminal residue;
and pharmaceutically active salts thereof.

This invention is also a pharmaceutical composition for inhibiting platelet aggregation and clot formation, which comprises a compound of formula (I) or (IA) or a compound of any of Examples 1-9, 11, 12, 15, 16, 19 or 21 (hereinafter 'a peptide of the invention') and a pharmaceutically acceptable carrier.

This invention is further a method for for inhibiting platelet aggregation in a mammal in need thereof, which comprises internally administering an effective amount of a peptide of the invention.

In another aspect, this invention provides a method for inhibiting reocclusion of an artery or vein in a mammal following thrombolysis, which comprises internally administering an effective amount of a fibrinolytic agent and a peptide of the invention. In combination with known fibrinolytics, such as streptokinase (SK), urokinase (UK), pro-urokinase (pUK) and tissue plasminogen activator (tPA) and variants or mutants thereof, these compounds are useful for inhibiting rethrombosis.

This invention is also a pharmaceutical composition for effecting thrombolysis and reperfusion, and inhibiting reocclusion in an artery or vein in a mammal, which comprises a fibrinolytic and a peptide of the invention in a pharmaceutical carrier.

Finally, this invention is a kit for use in a method for effecting thrombolytic therapy, which comprises, in a container, a fibrinolytic and a peptide of the invention.

### Detailed Description of the Invention

This invention discloses cyclic peptide-like compounds comprising the sequence Gly-Asp. The compounds of this invention inhibit platelet aggregation and are believed to interact with the GPIIb-IIIa receptor and other adhesion proteins.

In compounds of formula (I), B is suitably Arg or HArg, or an α-R' substituted derivative of Arg or HArg. B is preferably MeArg.

Preferably A is Dtc, Tpr or Pro, when A' is Asn, Gln, Ala or Abu.

Suitably Y is CONH₂ or CO₂H.

Suitably Z₂ is 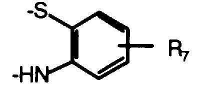 or Pcs.

Suitably Z₁ is 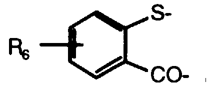 or Cys.

Suitably R₆ and R₇ are H.

Suitably A' is absent.

Suitably A is Sar or is absent.

In compounds of formula (IA), suitably, A and A' are absent, n is 2 and Z₁ and Z₂ are together a covalent bond.

Q is suitably Ser, (Me)Ser, Thr, Tyr, Phe or Nal, when n is 2. Q is preferably Phe.

The meaning of X in the formulae herein depicted is intended to denote the amino group of these amino acids. In like manner, Y refers to the substituted carboxyl group of these amino acids. It will be understood that when Z₁ and Z₂ are not an aryl moeity, they may have one or two chiral centers and that this invention includes each unique nonracemic compound which may be synthesized and resolved by conventional techniques.

When Z₁ or Z₂ are phenyl, a mercapto or alkylene group is in the 1 position, the amino/carboxyl group is in the 2 position, and they may be substituted in the 3, 4 or 5 position by R₆ or R₇. When Z₁ or Z₂ are naphthyl, the mercapto or alkylene group may be in the 1 or 2 position, the amino/carboxyl group bears an ortho orientation and they may be further substituted on any position of the naphthyl ring.

Het represents a substituted heterocycle. Representative heterocycles are pyridine, pyrrole, pyrrolidine, imidazole, triazole, thiophene, furan, and thiazole. Such heterocycles form a macrocyclic ring within the peptide via two ortho situated substituents. For example, Z₁ is a heterocyclic carboxylic acid, which is attached to the peptide through the carboxyl and to Z₂ via an ortho situated bridge as defined by L¹. Similarly, Z₂ may be a heterocyclic amine, which is attached to the peptide through the amine and to Z₁ via an ortho situated bridge defined as L².

C₁₋₄alkyl as applied herein is meant to include methyl, ethyl, n-propyl, isopropyl, n-butyl and isobutyl. Ar as applied herein means phenyl or phenyl substituted by one or two C₁₋₄alkyl, trifluoromethyl, C₁₋₄alkoxy, hydroxy,or halogen groups.

This invention includes compounds in which any of the peptide linkages, -CONH-, are replaced by an isosteric linkage. Examples of peptide isosteres are -NHCO-, -CH=CH-, -CH₂CH₂-, -COCH₂-, -COO-, -CHOHCH₂-, -CH₂NR₄-, -CSNH- and -CH₂S-.

Specific compounds of this invention are:
cyclo(S,S)-Mba-Arg-Gly-Asp-Cys-NH₂ (SEQ ID NO.1);
N^{α} -Ac-cyclo(S,S)-Cys-Arg-Gly-Asp-Man (SEQ ID NO.2);
cyclo (S,S)-Mba-MeArg-Gly-Asp-Man;
cyclo(S,S)-Mba-MeArg-Gly-Asp-Pcs-NH₂ (SEQ ID NO.3);
cyclo-(S,S)-Mba-Ser-Arg-Gly-Asp-Man (SEQ ID NO.4);
cyclo-(S,S)-Mba-Sar-MeArg-Gly-Asp-Man (SEQ ID NO.5);
cyclo-(S,S)-Mba-Arg-Gly-Asp-Man;
cyclo-(S,S)-Mba-D-MeArg-Gly-Asp-Man;
N^{α} Ac-cyclo-(S,S)-Cys-MeArg-Gly-Asp-(2R,3S)Pcs-NH₂ (SEQ ID NO.6);
N^{α}Ac-cyclo-(S,S)-Cys-MeArg-Gly-Asp-(2R,3R)Pcs-NH₂ (SEQ ID NO.7);
N^{α}Ac-cyclo-(S,S) Cys-Arg-Gly-Asp-Ser-MeArg -Gly-Asp-Ser-Cys-NH₂ (SEQ ID NO.10) ;
N^{α}Ac-cyclo-(S,S)-Cys-MeArg-Gly-Asp-Ser-Arg -Gly-Asp-Ser-Cys-NH₂ (SEQ ID NO.11);
cyclo(1^{α},6^{γ})-Gly-MeArg-Gly-Asp-Ser-Glu-NH₂ (SEQ ID NO.14);
cyclo-(1,8)-MeArg-Gly-Asp-Phe-Arg-Gly-Asp-Phe (SEQ ID NO.16);

Preferred compounds of this invention are:
cyclo-(S,S)-Mba-MeArg-Gly-Asp-Man;
cyclo-(S,S)-Mba-Sar-MeArg-Gly-Asp-Man (SEQ ID NO.5);
N^{α}Ac-cyclo-(S,S)-Cys-MeArg-Gly-Asp-Pcs (SEQ ID NO.18); and
cyclo(1,8)-MeArg-Gly-Asp-Phe-Arg-Gly-Asp-Phe (SEQ ID NO.16).

The nomenclature commonly used in the art is used herein to describe the peptides.

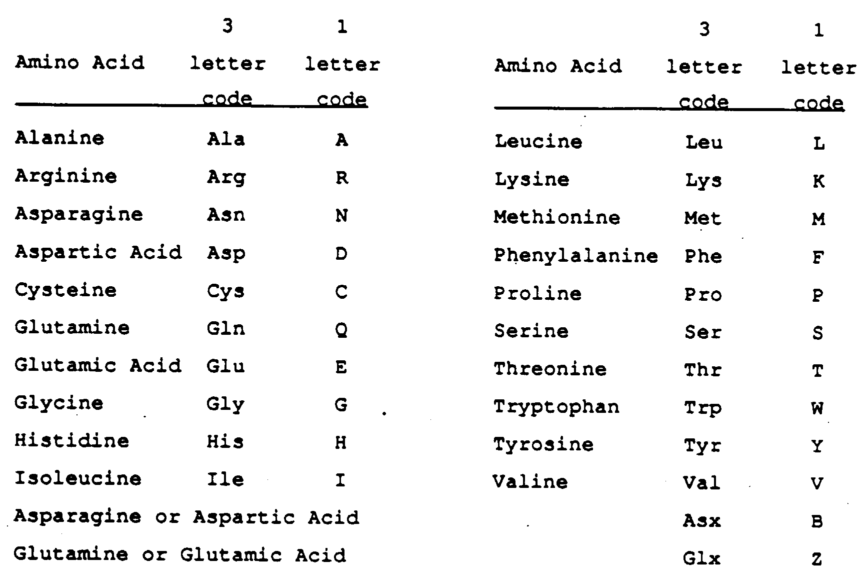

In accordance with conventional representation, the amino terminus is on the left and the carboxy terminus is on the right. Unless specified otherwise, all chiral amino acids (AA) are assumed to be of the L-absolute configuration. Pen refers to L-penicillamine or β,β dimethyl cysteine, APmp refers to 2-amino-3,3-cyclopentamethylene-3-mercaptopropionic acid, Dtc refers to 5,5-dimethylthiazolidine-4-carboxylic acid, Tpr refers to thiazolidine-4-carboxylic acid, Mpa refers to 3-mercaptopropionic acid, Pmp refers to 3,3-cyclopentamethylene-3-mercaptopropionic acid, Mdp refers to 3-mercapto-3-methylbutanoic acid, Pcs refers to 3-phenyl cysteine racemic in the 3 position, (3S)Pcs refers to (2R,3S)-3-phenylcysteine, (3R)Pcs refers to (2R,3R)-3-phenylcysteine, Man refers to 2-mercapto-aniline, Mba refers to 2-mercapto-benzoic acid, HArg refers to homoarginine, NArg refers to norarginine, (Me₂)Arg refers to N', N"-dimethyl arginine, (Et₂)Arg refers to N', N"-diethyl arginine, Nva refers to norvaline, Nle refers to norleucine, α-MeAsp refers to N^{α}-methyl aspartic acid, Nal refers to beta-2-naphthyl alanine, Phg refers to phenylglycine, HPhe refers to homophenylalanine, Abu refers to 2-amino butyric acid, (Alk)Tyr refers to O-C₁₋₄alkyl-tyrosine, (Alk)Ser refers to O-C₁₋₄alkyl-serine, (Alk)Thr refers to O-C₁₋₄alkyl-threonine, (Alk)Cys refers to S-C₁₋₄alkyl-cysteine, (Alk)Pen refers to S-C₁₋₄alkyl-penicillamine, (4'W)Phe refers to phenylalanine substituted in the 4 position of the phenyl ring by W, t-Bu refers to the tertiary butyl radical, Boc refers to the t-butyloxycarbonyl radical, Fmoc refers to the fluorenylmethoxycarbonyl radical, Ph refers to the phenyl radical, Cbz refers to the carbobenzyloxy radical, BrZ refers to the o-bromobenzyloxycarbonyl radical, ClZ refers to the o-chlorobenzyloxycarbonyl radical, Bzl refers to the benzyl radical, 4-MBzl refers to the 4-methyl benzyl radical, Ac refers to acetyl, Alk refers to C₁₋₄ alkyl, Ph refers to phenyl, Nph refers to 1- or 2-naphthyl, cHex refers to cyclohexyl, DCC refers to dicyclohexylcarbodiimide, DMAP refers to dimethylaminopyridine, DIEA refers to diisopropylethyl amine, EDC refers to N-ethyl-N'(dimethylaminopropyl)carbodiimide, HOBT refers to 1-hydroxybenzotriazole, THF refers to tetrahydrofuran, DMF refers to dimethyl formamide, PPA refers to 1-propanephosphonic acid cyclic anhydride, DPPA refers to diphenylphosphoryl azide, BOP refers to benzotriazol-1-yloxy-tris(dimethylamino)phosphonium hexafluorophosphate, HF refers to hydrofluoric acid and TFA refers to trifluoroacetic acid.

Coupling reagents as used herein denote reagents which may be used to form peptide bonds. Typical coupling reagents are carbodiimides, activated anhydrides and esters and acyl halides. Reagents such as EDC, DCC, DPPA, PPA, BOP reagent, HOBT, N-hydroxysuccinimide and oxallyl chloride are typical.

α-R' substituted derivatives of the amino acids of this invention, which may be denoted as (α-R')AA, indicate amino acids which are mono-substituted on the α-amino group by R', wherein R' is Alk or benzyl. R' is preferably methyl. N^{α} -methyl arginine and N^{α}-methyl glycine, which are (α-Me)Arg and (α-Me)Gly respectively, are also denoted herein as MeArg and Sar (sarcosine) in accordance with past conventional notation. All other N-α-substituted amino acids will carry the designation α- in their representation. Thus, amino acids which may be alkylated upon a mercaptan, guanidino or hydroxyl group, such as Tyr, Ser, Thr, Cys or Pen, are distinguished by an absence of this designation. Thus, (α-Me)Ser is N^{α}-methyl serine, (Me)Ser is O-methyl serine, (α-Me,Et)Ser is N^{α}-methyl, O-ethyl serine and (α-Me,Et₂)Arg is N^{α}-methy-N',N"-diethyl arginine.

The peptides are prepared preferably by the solid phase technique of Merrifield (J. Am. Chem. Soc., 85, 2149 (1964)), although solution methods known to the art may be successfully employed. A combination of solid phase and solution synthesis may be used, as in a convergent synthesis in which di-, tri-, tetra-, or penta-peptide fragments may be prepared by solid phase synthesis and either coupled or further modified by solution synthesis. The methods of peptide synthesis generally set forth by Ali et al. in J. Med. Chem., 29, 984 (1986) and J. Med. Chem., 30, 2291 (1987) were employed to produce most of the peptides of this invention and are incorporated herein by reference.

The reactive functional groups of the sidechains of each amino acid or peptide are suitably protected as known in the peptide art. For example, the Boc, Cbz or Fmoc group may be used for protection of an amino group, especially an α-amino group. The Boc group is generally preferred for protection of the α-amino group. A t-Bu, cHex or benzyl ester may be used for the protection of the side chain carboxyl of Asp or Glu. A benzyl group or suitably substituted benzyl group is used to protect the mercapto group of cysteine, or other thiol containing residues; or the hydroxyl of serine or threonine. The tosyl group may be used for protection of the imidazolyl group of His, and tosyl or nitro group for protection of the guanidino nitrogen of Arg. A suitably substituted carbobenzyloxy group or benzyl group may be used for the hydroxyl group of Tyr, Ser or Thr, or the ε-amino group of lysine. The phthalamido group may also be used for the protection of the ε-amino group of lysine. Suitable substitution of the carbobenzyloxy or benzyl protecting groups is ortho and/or para substitution with chloro, bromo, nitro or methyl, and is used to modify the reactivity of the protective group. Cysteine and other sulfur-containing amino acids may also be protected by formation of a disulfide with a thioalkyl or thioaryl group. Except for the Boc group, the protective groups are, most conveniently, those which are not removed by mild acid treatment. These protective groups are removed by such methods as catalytic hydrogenation, sodium in liquid ammonia or HF treatment, as known in the art.

If solid phase methods are used, the peptide is built up sequentially starting from the carboxy terminus and working toward the amino terminus of the peptide. Solid phase synthesis is begun by covalently attaching the C terminus of a protected amino acid to a suitable resin, such as a benzhydrylamine resin (BHA), methylbenzhydrylamine resin (MBHA), chloromethyl resin (CMR), hydroxymethyl resin (HMR) or SASRIN resin, as is generally set forth in U.S. Patent No. 4,244,946. A BHA or MBHA support resin is used if the carboxy terminus of the product peptide is to be a carboxamide. A CMR support is generally used if the carboxy terminus of the product peptide is to be a carboxyl group, although this may also be used to produce a carboxamide or ester.

Once the first protected amino acid (AA) has been coupled to the desired resin, the amino group is deprotected by mild acid treatment, and the free carboxyl of the second protected AA is coupled to this amino group. This process is carried out sequentially, without isolation of the intermediate, until the desired peptide has been formed. The completed peptide may then be deblocked and/or split from the carrying resin in any order.

Treatment of a CMR supported peptide with alkali in aqueous alcohol splits the peptide from the resin and produces the carboxy terminal amino acid as a carboxylic acid. Treatment of a CMR supported peptide with ammonia or alkyl amines in an alcoholic solvent provides a carboxamide or alkyl carboxamide at the carboxy terminus.

If an ester is desired, the CMR resin may be treated with an appropriate alcohol, such as methyl, ethyl, propyl, butyl or benzyl alcohol, in the presence of triethylamine to cleave the peptide from the resin and produce the ester directly.

Esters of the peptides of this invention may also be prepared by conventional methods from the carboxylic acid precursor. Typically, the carboxylic acid is treated with an alcohol in the presence of an acid catalyst. Alternatively, the carboxylic acid may be converted to an activated acyl intermediate, such as an acid halide, and treated with an alcohol, preferably in the presence of a base.

Methods of producing C-terminal esters of the peptides without esterification of the side chain carboxyl group of aspartic acid are slightly more elaborate, but are well known to those skilled in the art of peptide synthesis. For example, the synthesis is begun with an ester of the C terminal amino acid, or of a dipeptide, and coupled via solution phase synthesis to an appropriately side-chain-protected aspartic acid residue. The side chain carboxyl group is then selectively deprotected and coupled to a chloromethyl resin (CMR). The amino group is liberated and solid phase peptide synthesis is employed. Subsequent cleavage from the resin, using HF, produces the desired side chain carboxylic acid, whilst the carboxy terminus of the peptide remains as an ester. In a similar manner, if one begins the synthetic sequence with the alkyl amide of an appropriately protected amino acid or dipeptide, one obtains the corresponding C-terminal alkyl amide of the peptide.

For producing esters and substituted amides in such a process, suitable protecting groups for the 4-carboxyl group of aspartic acid are benzyl esters and halogen- or alkyl-substituted benzyl esters. When the amino group is protected by the Boc group, the benzyl ester protecting group may be selectively removed by hydrogenation and coupled to a CMR support.

If Z₂ possesses no carboxylic acid moeity or there are benzyl or substituted benzyl protecting groups (such as for the hydroxyl, thiol or amino group) on the amino acid (or dipeptide) which is to be coupled to the aspartic acid prior to attachment to the resin, a t-butyl ester or other acid labile group is suitable for protecting the side-chain carboxyl of the aspartic acid. In this case the amino group of the aspartic acid is protected by a base labile group, such as the fluorenylmethoxycarbonyl moiety (Fmoc). After solution phase coupling of the aspartic acid to an aniline, amine or amino acid (or dipeptide), selective deprotection of the t-butyl ester is accomplished by mild acid hydrolysis and the side chain carboxyl is coupled to the resin by conventional methods. The fluorenylmethoxycarbonyl group is then removed by mild base for subsequent solid phase peptide synthesis. When the terminal residue, Z₂, is a substituted or unsubstituted o-mercapto aryl amine, which bears no carboxyl substituent, this method is particularly effective.

The preferred method for cleaving a peptide from the support resin is to treat the resin supported peptide with anhydrous HF in the presence of a suitable cation scavenger, such as anisole or dimethoxy benzene. This method simultaneously removes all protecting groups, except a thioalkyl group protecting sulfur, and splits the peptide from the resin. Peptides hydrolyzed in this way from the CMR are carboxylic acids, those split from the BHA resin are obtained as carboxamides.

The cyclic disulfide compounds of formula (I) are produced from a corresponding linear peptide of formula(II), 
S-T^1
|
Z_1-A'-A-(B-Gly-Asp-Q)_n-M-Z_2

S-T^2
|

(II)
 wherein A', A, B, Q, M, and n, are as defined hereinbefore for structure (I), Z₁ is 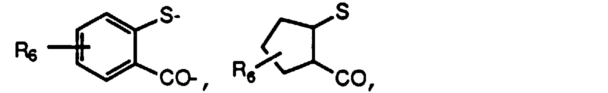 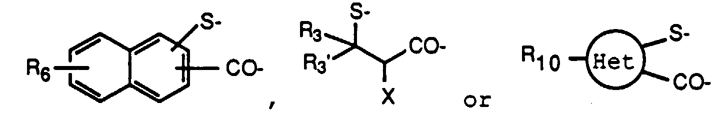 and Z₂ is 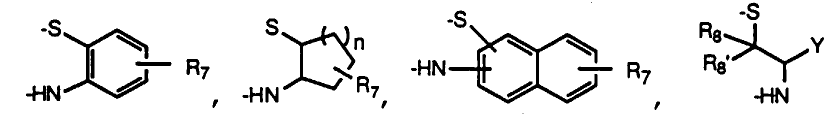 or 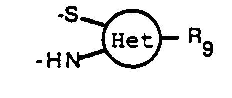 with any chemically reactive centers optionally protected as previously described, and the sulfur moeity of Z₁ or Z₂ is substituted by T¹ or T². T¹ and T² are displaceable groups such as a thioalkyl, thioaryl group, substituted benzyl group or hydrogen. Examples of suitable displaceable groups are hydrogen, C₁₋₄alkylthio, especially ethylthio, benzyl and the 4-methyl benzyl group. Preferably T1 and T2 are both hydrogen, or one of T¹ and T² is hydrogen and the other is C₁₋₄alkylthio.

Formation of the disulfide bond may be accomplished by either one of two general methods. If the sulfur-containing amino acids of the linear peptide are protected differently, in such a manner as to allow formation of a mono mercaptan, cyclization may be effected by base catalyzed nucleophilic displacement of the protecting group of the second sulfur-containing amino acid. Groups which are especially useful as displaceable protecting groups are thioalkyl or thioaryl groups. Exemplary of this method is the protection of one sulfur-containing amino acid by the thioethyl group, and protection of the second by a substituted benzyl group. Deprotection of such a peptide by HF removes the benzyl group from one amino acid, while leaving the second protected as an ethyl disulfide. Stirring this mercapto/disulfide in dilute solution at a pH of about 7 to 8 effects displacement of the thioethyl group and cyclization of the linear peptide.

If the corresponding linear peptide of formula (II) is completely deprotected and produced as a dimercaptan, any oxidizing agent known to the art to be capable of converting a dimercaptan to a disulfide may be used. Exemplary of such agents are an alkali metal ferricyanide, especially potassium or sodium ferricyanide, oxygen gas, diiodomethane or iodine. Thus, treatment of a compound of formula (II) with an oxidant causes cyclization of the compound. The reaction is conducted in a suitable inert solvent, such as aqueous methanol or water, at temperatures from about 0 to about 40° C, under high dilution. The pH is usually maintained at about 7 to about 8. Cyclization may be performed upon the peptide while it is still attached to the support resin or while other functional groups are still protected, but it is preferrably performed on the deprotected free peptide.

Accordingly, this invention is also a process for preparing a compound of formula (III), 
S                                    S
|                                    |
Z1-A'-A-(B-Gly-Asp-Q)n-M-Z2

(III)
 wherein A', A, B, Q, M, Z₁, Z₂ and n are as previously defined for formula (I), which comprises,
a) oxidatively cyclizing a compound of the formula (II), 
S-T1
                                        S-T2
|
                                        |
Z1-A'-A-(B-Gly-Asp-Q)n-M-Z2

(II)
 wherein A', A, B, Q, M, Z₁, Z₂ and n are as previously defined for formula (I), and T¹ and T² are H, or
b) nucleophilically cyclizing a compound of formula (II) by treating with base, wherein A', A, B, Q, M, Z₁, Z₂ and n are as previously defined for formula (I), and one of T¹ and T² is a displaceable group and the other is H.

Another group of compounds of formula (IA) are homodetic peptides wherein Z₁ and Z₂ are, taken together, a covalent bond. These peptides are prepared from the linear peptides of formula (IV), wherein A', A, B, Q, M and n 
H-[A'-A-(B-Gly-Asp-Q-)n-M]-OH
(IV)
 are as hereinbefore defined for formula (IA). These linear peptides are prepared in a manner to liberate and cyclize the terminal amino group and terminal carboxyl group of the peptide while the side chain carboxyl of the Asp remains protected. Cyclization may then be effected by common peptide bond forming reagents, such as carbodiimides or activated anhydrides. Diphenyl phosphoryl azide, the BOP reagent, 1-propanephosphonic acid cyclic anhydride and DCC/HOBT are examples of such reagents. It will be apparent that the terminal amino group of peptide (IV), denoted as H-[HN-AA-], and the terminal carboxyl group, denoted as [-AA-CO]-OH, may be attached to any residue of the peptide (i.e. A', A, B, Gly, Asp, Q or M), since the formation of any peptide bond in the cyclic peptides may be effected in the last step to prepare the same final compound.

When Asp or Glu are present in the peptide, there is a possibility of cyclization through either the sidechain β-carboxy (Asp) or γ-carboxy (Glu) group or the terminal carboxy group of the amino acid. Cyclization through the terminal carboxy group is preferred for Asp. Cyclization through the γ-carboxy group is suitable for Glu. Methods common to the art, as more fully illustrated herein, are available for cyclizing through either carboxyl group.

Acccordingly, this invention is also a process for preparing a compound of formula (IA)
which comprises,
i) cyclizing with a coupling reagent a compound of the formula: H-[A'-A-(B-Gly-Asp-Q-)n-M]-OH when Z₁ and Z₂ are, together, a covalent bond;
   wherein H- and -OH represent the amino and carboxyl residues of any two adjacent residues in the cyclic peptide, and A', A, B, Q, M and n are as defined above with any reactive groups optionally protected, and
ii) removing any protecting groups.

Modification of the terminal amino group of the peptide is accomplished by alkylation or acetylation as is generally known in the art. These modifications may be carried out upon the amino acid prior to incorporation into the peptide, or upon the peptide after it has been synthesized and the terminal amino group liberated, but before the protecting groups have been removed.

Typically, acetylation is carried out upon the free amino group using the acyl halide, or anhydride, of the corresponding alkyl or aryl acid, in the presence of a tertiary amine. Mono-alkylation is carried out most conveniently by reductive alkylation of the amino group with an appropriate aliphatic aldehyde or ketone in the presence of a mild reducing agent, such as lithium or sodium cyanoborohydride. Dialkylation may be carried by treating the amino group with an excess of an alkyl halide in the presence of a base.

Solution synthesis of peptides is accomplished using conventional methods used to form amide bonds. Typically, a protected Boc-amino acid which has a free carboxyl group is coupled to a protected amino acid which has a free amino group using a suitable carbodiimide coupling agent, such as N, N' dicyclohexyl carbodiimide (DCC), optionally in the presence of catalysts such as 1-hydroxybenzotriazole (HOBT) and dimethylamino pyridine (DMAP). Other methods, such as the formation of activated esters, anhydrides or acid halides, of the free carboxyl of a protected Boc-amino acid, and subsequent reaction with the free amine of a protected amino acid, optionally in the presence of a base, are also suitable. For example, a protected Boc-amino acid or peptide is treated in an anhydrous solvent, such as methylene chloride or tetrahydrofuran(THF), in the presence of a base, such as N-methyl morpholine, DMAP or a trialkylamine, with isobutyl chloroformate to form the "activated anhydride", which is subsequently reacted with the free amine of a second protected amino acid or peptide. The peptide formed by these methods may be deprotected selectively, using conventional techniques, at the amino or carboxy terminus and coupled to other peptides or amino acids using similar techniques.

The α-R' substituted derivatives of the amino acids of this invention, which includes derivatives of Arg, HArg, (Me₂)Arg, (Et₂)Arg, Ala, Gly, His, Abu, Tyr, (Alk)Tyr, Phe, (4'W)Phe, HPhe, Phg, Trp, His, Ser, (Alk)Ser, Thr, (Alk)Thr, Cys, (Alk)Cys, Pen, (Alk)Pen, Ala, Val, Nva, Met, Leu, Ile, Nle and Nal, are prepared by methods common to the chemical art. The R' substituent may be Alk, as hereinbefore defined, or benzyl. Representative methods for preparing these derivatives are disclosed in U.S. Patent No. 4,687,758; Cheung et al., Can. J. Chem., 55, 906 (1977); Freidinger et al., J. Org. Chem., 48, 77, (1982); and Shuman et al., Peptides: Proceedings of the 7th American Peptide Symposium, Rich, D., Gross, E., Eds, Pierce Chemical Co., Rockford, Ill., 617 (1981), which are incorporated herein by reference. Typically, a solution of the Cbz- or Boc-amino acid in DMF/THF is condensed with an appropriate alkyl halide, such as methyl or ethyl iodide, in the presence of a base, such as sodium hydride or potassium hydride. Optionally, a crown ether, such as 18-crown-6 with potassium hydride, may be added to facilitate the reaction. Generally, in this process and those that follow, if the amino acid bears a functional group such as a hydroxyl, mercaptan, amino, guanidino, indolyl or imidazolyl group, these groups are protected as hereinbefore described. Thus, Boc-Tyr(Bzl) is treated with sodium hydride and methyl iodide in THF/DMF solution at O°C and stirred at room temperature for 24 hrs. to yield Boc-(α-Me)Tyr(Bzl).

Alternately, the free amine of the amino acid is reacted with an appropriate aldehyde, such as acetaldehyde or benzaldehyde, in the presence of a reducing agent, such as sodium cyanoborohydride, to effect mono-alkylation. This process is especially useful for preparing α-benzyl amino acids. α-Benzylated amino acids may also be used as intermediates to prepare α-methyl amino acids. For example, α-methyl arginine is prepared in three steps by 1.) reacting Arg(Tos) with benzaldehyde and sodium cyanoborohydride in a methanol solution to yield (α-Bzl)Arg(Tos); 2.) reducing the benzylated product with formaldehyde/formic acid solution to yield (α-Bzl, α-Me)Arg(Tos); and 3.) liberating the benzyl group by catalytic hydrogenation (5% Pd/C in glacial acetic acid/HCl) to yield MeArg(Tos).

α-R' substituted derivatives of amino acids may also be prepared by reduction of oxazolidinones prepared from the Fmoc- or Cbz-amino acids. Typically, an Fmoc- or Cbz-amino acid is heated with an appropriate aldehyde such as acetaldehyde or benzaldehyde, in the presence of toluenesulfonic acid, in toluene solution to produce a 2-substituted 5-oxo-oxazolidine. Reduction of this oxazolidinone with triethylsilane and TFA in chloroform solution affords the Cbz- or Fmoc-α substituted amino acid directly. It will be appreciated by those skilled in the art that when formaldehyde is used, the oxazolidinone is unsubstituted in the 2-position and α-methyl amino acids are produced.

Acid addition salts of the peptides are prepared in a standard manner in a suitable solvent from the parent compound and an excess of an acid, such as hydrochloric, hydrobromic, sulfuric, phosphoric, acetic, maleic, succinic or methanesulfonic. The acetate salt form is especially useful. Certain of the compounds form inner salts or zwitterions which may be acceptable. Cationic salts are prepared by treating the parent compound with an excess of an alkaline reagent, such as a hydroxide, carbonate or alkoxide, containing the appropriate cation; or with an appropriate organic amine. Cations such as Li+, Na+, K+, Ca++, Mg++ and NH₄+ are specific examples of cations present in pharmaceutically acceptable salts.

This invention provides an pharmaceutical composition which comprises a peptide of the invention and a pharmaceutically acceptable carrier. Pharmaceutical compositions of the peptides prepared as hereinbefore described and other peptide or polypeptide derivatives of fibronectin, fibrinogen or Von Willebrand's factor, may be formulated as solutions or lyophilized powders for parenteral administration. Powders may be reconstituted by addition of a suitable diluent or other pharmaceutically acceptable carrier prior to use. The liquid formulation is generally a buffered, isotonic, aqueous solution. Examples of suitable diluents are normal isotonic saline solution, standard 5% dextrose in water or buffered sodium or ammonium acetate solution. Such formulation is especially suitable for parenteral administration, but may also be used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride or sodium citrate.

Alternately, these peptides may be encapsulated, tableted or prepared in a emulsion or syrup for oral administration. Pharmaceutically acceptable solid or liquid carriers may be added to enhance or stabilize the composition, or to facilitate preparation of the composition. Solid carriers include starch, lactose, calcium sulfate dihydrate, terra alba, magnesium stearate or stearic acid, talc, pectin, acacia, agar or gelatin. Liquid carriers include syrup, peanut oil, olive oil, saline and water. The carrier may also include a sustained release material such as glyceryl monostearate or glyceryl distearate, alone or with a wax. The amount of solid carrier varies but, preferably, will be between about 20 mg to about 1 g per dosage unit. The pharmaceutical preparations are made following the conventional techniques of pharmacy involving milling, mixing, granulating, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly p.o. or filled into a soft gelatin capsule.

For rectal administration, the peptides of this invention may also be combined with excipients such as cocoa butter, glycerin, gelatin or polyethylene glycols and molded into a suppository.

This invention also provides a method of inhibiting platelet aggregation and clot formation in a mammal, especially a human, which comprises the internal administration of a peptide of the invention and a pharmaceutically acceptable carrier. Indications for such therapy include acute myocardial infarction (AMI), deep vein thrombosis, pulmonary embolism, dissecting anurysm, transient ischemia attack (TIA), stroke and other infarct-related disorders, and unstable angina. Chronic or acute states of hyper-aggregability, such as disseminated intravascular coagulation (DIC), septicemia, surgical or infectious shock, post-operative and post-partum trauma, cardiopulmonary bypass surgery, incompatible blood transfusion, abruptio placenta, thrombotic thrombocytopenic purpura (TTP), snake venom and immune diseases, are likely to be responsive to such treatment. In addition, the peptides of this invention may be used in a method for the prevention of metastatic conditions.

The peptide is administered either orally or parenterally to the patient, in a manner such that the concentration of drug in the plasma is sufficient to inhibit platelet aggregation. The pharmaceutical composition containing the peptide is administered at a dose between about .2 to about 50 mg/kg in a manner consistent with the condition of the patient. For acute therapy, parenteral administration is preferred. For persistant states of hyperaggregability, an intravenous infusion of the peptide in 5% dextrose in water or normal saline is most effective, although an intramuscular bolus injection may be sufficient.

For chronic, but noncritical, states of platelet aggregability, oral administration of a capsule or tablet, or a bolus intramuscular injection is suitable. The peptide is administered one to four times daily at a level of about .4 to about 50 mg/kg. to achieve a total daily dose of about .4 to about 200 mg/kg/day.

This invention further provides a method for inhibiting the reocclusion of an artery or vein following fibrinolytic therapy, which comprises internal administration of a peptide of the invention and a fibrinolytic agent. It has been found that administration of an peptide in fibrinolytic therapy either prevents reocclusion completely or prolongs the time to reocclusion.

When used in the context of this invention the term fibrinolytic agent is intended to mean any compound, whether a natural or synthetic product, which directly or indirectly causes the lysis of a fibrin clot. Plasminogen activators are a well known group of fibrinolytic agents. Useful plasminogen activators include, for example, anistreplase, urokinase (UK), pro-urokinase (pUK), streptokinase (SK), tissue plasminogen activator (tPA) and mutants, or variants, thereof, which retain plasminogen activator activity, such as variants which have been chemically modified or in which one or more amino acids have been added, deleted or substituted or in which one or more or functional domains have been added, deleted or altered such as by combining the active site of one plasminogen activator with the fibrin binding domain of another plasminogen activator or fibrin binding molecule. Other illustrative variants include tPA molecules in which one or more glycosylation sites have been altered. Preferred among plasminogen activators are variants of tPA in which the primary amino acid sequence has been altered in the growth factor domain so as to increase the serum half-life of the plasminogen activator. tPA Growth factor variants are disclosed, e.g., by Robinson et al., EP-A 0 297 589 and Browne et al., EP-A 0 240 334 and in GB 8815135.2. Other variants include hybrid proteins, such as those disclosed in EP 0 028 489, EP 0 155 387 and EP 0 297 882, all of which are incorporated herein by reference. Anistreplase is a preferred hybrid protein for use in this invention. Fibrinolytic agents may be isolated from natural sources, but are commonly produced by traditional methods of genetic engineering.

Useful formulations of tPA, SK, UK and pUK are disclosed, for example, in EP-A 0 211 592 (U.S. Ser. No. 890,432), German Patent Application No. 3032606, EP-A 0 092 182 and U.S. Patent 4,568,543, all of which are incorporated herein by reference. Typically the fibrinolytic agent may be formulated in an aqueous, buffered, isotonic solution, such as sodium or ammonium acetate or adipate buffered at pH 3.5 to 5.5. Additional excipients such as polyvinyl pyrrolidone, gelatin, hydroxy cellulose, acacia, polyethylene, glycol, mannitol and sodium chloride may also be added. Such a composition can be lyophilized.

The pharmaceutical composition may be formulated with both the peptide and fibrinolytic in the same container, but formulation in different containers is preferred. When both agents are provided in solution form they can be contained in an infusion/injection system for simultaneous administration or in a tandem arrangement.

Indications for such therapy include myocardial infarction, deep vein thrombosis, pulmonary embolism, stroke and other infarct-related disorders. The peptide is administered just prior to, at the same time as, or just after parenteral administration of tPA or other fibrinolytic agent. It may prove desirable to continue treatment with the peptide for a period of time well after reperfusion has been established to maximally inhibit post-therapy reocclusion. The effective dose of tPA, SK, UK or pUK may be from .5 to 5 mg/kg and the effective dose of the peptide may be from about .1 to 25 mg/kg.

For convenient administration of the inhibitor and the fibrinolytic agent at the same or different times, a kit is prepared, comprising, in a single container, such as a box, carton or other container, individual bottles, bags, vials or other containers each having an effective amount of the inhibitor for parenteral administration, as described above, and an effective amount of tPA, or other fibrinolytic agent, for parenteral administration, as described above. Such kit can comprise, for example, both pharmaceutical agents in separate containers or the same container, optionally as lyophilized plugs, and containers of solutions for reconstitution. A variation of this is to include the solution for reconstitution and the lyophilized plug in two chambers of a single container, which can be caused to admix prior to use. With such an arrangement, the fibrinolytic and the peptide may be packaged separately, as in two containers, or lyophilized together as a powder and provided in a single container.

When both agents are provided in solution form, they can be contained in an infusion/injection system for simultaneous administration or in a tandem arrangement. For example, the platelet aggregation inhibitor may be in an i.v. injectable form, or infusion bag linked in series, via tubing, to the fibrinolytic agent in a second infusion bag. Using such a system, a patient can receive an initial bolus-type injection or infusion, of the peptide inhibitor followed by an infusion of the fibrinolytic agent.

The pharmacological activity of the peptides was assessed by the following tests:

### In Vivo Inhibition of Platelet Aggregation

In vivo inhibition of thrombus formation is demonstrated by recording the systemic and hemodynamic effects of infusion of the peptides into anesthetized dogs according to the methods described in Aiken et al., Prostaglandins, 19, 629-43 (1980).

### Inhibition of Platelet Aggregation

Blood was collected (citrated to prevent coagulation) from, naive, adult mongrel dogs. Platelet rich plasma, PRP, was prepared by centrifugation at 150 x g for 10 min at room temperature. Washed platelets were prepared by centrifuging PRP at 800 x g for 10 min. The cell pellet thus obtained was washed twice in Tyrode's buffer (pH 6.5) without Ca⁺⁺ and resuspended in Tyrode's buffer (pH 7.4) containing 1.8 mM Ca⁺⁺ at 3 x 10⁵ cells/ml. Peptides were added 3 min prior to the agonist in all assays of platelet aggregation. Final agonist concentrations were 0.1 unit/ml thrombin and 2 mM ADP (Sigma). Aggregation was monitored in a Chrono-Log Lumi-Aggregometer. Light transmittance 5 min after addition of the agonist was used to calculate percent aggregation according to the formula % aggregation = [(90-CR) ö (90-10)] x 100, where CR is the chart reading, 90 is the baseline, and 10 is the PRP blank reading. IC₅₀'s were determined by plotting [% inhibition of aggregation] vs. [concentration of peptide]. Peptides were assayed at 200 mM and diluted sequentially by a factor of 2 to establish a suitable dose response curve.

To assess the stability of the peptide to plasma proteases, the peptides were incubated for 3 hrs. (rather than 3 min) in the PRP prior to addition of the agonist.

The compounds of Examples 1-21 showed an IC₅₀ for the aggregation of dog platelets stimulated by ADP of between about 0.1 and 50 µM. Preferred compounds have an IC₅₀ of less than 10 µM.

The examples which follow are intended to in no way limit the scope of this invention, but are provided to illustrate how to make and use the compounds of this invention. Many other embodiments will be readily apparent and available to those skilled in the art.

### EXAMPLES

In the examples which follow all temperatures are in degrees centigrade. Amino acid analysis was performed upon a Dionex autoion 100. Analysis for peptide content is based upon amino acid analysis. Mass spectra were performed upon a VG Zab mass spectrometer using fast atom bombardment. EM silica gel thin layer (.25 mm) plates were used for thin layer chromatography. ODS refers to an octadecylsilyl silica gel chromatographic support. The abbreviations used to represent the eluent composition are n-BuOH: n-butanol, HOAc: acetic acid, H₂O: water, EtOAc: ethyl acetate, i-ProH: isopropanol, P: pyridine and CA: chloroacetic acid. HPLC was performed upon a Beckman 344 gradient chromatography system with a CRIB recording integrator in either an isocratic or continuous gradient mode. Solid phase peptide synthesis was performed using an automated Beckman 990 synthesizer. Where indicated, the purity of the peptide is based upon integration of the HPLC chromatogram. MeArg was prepared by the method disclosed by Ali et al., in U.S. Patent 4,687,758 (1987).

### Example 1

### Preparation of cyclo(S,S)-(2-mercapto)benzoyl-arginyl-glycyl-aspartyl-cysteineamide [cyclo-(S,S)-Mba-Arg-Gly-Asp-Cys-NH₂] (SEQ ID NO: 1)

### General procedure for solid phase peptide synthesis on benzhydylamine resin

Peptide amides were synthesized by solid phase peptide synthesis using benzhydrylamine resin as the support. Protected amino acids were added sequentially starting from the carboxyl terminus until the desired sequence was obtained. The t-butyloxycarbonyl (Boc) group was used for protection of the alpha-amino group. Side chain functional groups were protected as follows:; arginine and histidine, tosyl (Tos); cysteine and 3-phenylcysteine, p-methylbenzyl (MBzl) or ethylthio (SEt); serine and threonine, benzyl ether (Bzl); lysine, p-chlorocarbobenzoxy (ClZ); glutamic acid and aspartic acid, benzyl ester (OBzl) or cyclohexyl ester (O-cHex); tyrosine, p-bromocarbobenzoxy (BrZ). Removal of the Boc group was accomplished by treatment with 50% trifluoroacetic acid (TFA) in methylene chloride. Neutralization of the amine-TFA salt was accomplished by treatment with 7% diisopropylethylamine (DIEA) in methylene chloride. Amino acids were coupled to the growing peptide using 3 equivalents of Boc-amino acid and 3 equivalents of 1-hydroxybenzotriazole (HOBt) in DMF and 3 equivalents of dicyclohexylcarbodiimide (DCC) in methylene chloride. Completeness of coupling was checked by ninhydrin test and couplings were repeated as necessary. The general protocol is given below.

| | | |
|---|---|---|
| 1. | Wash with methylene chloride | 1 x 1 min |
| 2. | Wash with 50% TFA | 1 x 1 min |
| 3. | Deblock with 50% TFA | 1 x 20 min |
| 4. | Wash with methylene chloride | 6 x 1 min |
| 5. | Neutralize with 7% DIEA | 3 x 2 min |
| 6. | Wash with methylene chloride | 4 x 1 min |
| 7. | Wash with dimethylformamide | 2 x 1 min |
| 8. | Boc-AA + HOBt in DMF | do not drain |
| 9. | DCC in methylene chloride | 2 h |
| 10. | Wash with dimethylformamide | 2 x 1 min |
| 11. | Wash with methylene chloride | 3 x 1 min |

For attachment of the first (C-terminal) residue to the BHA resin, the synthesis was begun at step 5. For all subsequent amino acids, the synthesis was begun at step 1.

### a) 2-S-ethylmercaptobenzoic acid

To argon purged hexane (50 mL), ethanethiol (3.7 mL, 50 mmol) and sulfuryl chloride (4.0 mL, 50 mmol) were added and the solution stirred for 30 min. Toluene (100 mL) was added followed immediately by 2-mercaptobenzoic acid (7.71 g, 50 mmol). The reaction mixture was stirred at room temperature for 3 h and the solid product precipitated out. The solid was filtered, dried and chromatographed (silica gel, ethyl acetate) to yield the titled compound as tan solid (3.8 g, 36%).

### b) cyclo-(S,S)-Mba-Arg-Gly-Asp-Cys-NH₂ (SEQ ID NO: 1)

The protected peptide-resin intermediate, Mba(SEt)-Arg(Tos)-Gly-Asp(O- Bzl)-Cys(4-MBzl)-MBHA, was synthesized by the solid-phase method on 4- methylbenzhydrylamine resin, using an automated Beckman 990 synthesizer on 1.0 mmol scale. All of the amino acids were protected as t-butyloxycarbonyl on the amino group, and were coupled sequentially using N,N-dicyclohexylcarbodiimide/1-hydroxybenzotriazole (DCC/HOBt) in the manner set forth by Ail et al. in J. Med. Chem., 30, 2291 (1987) and J. Med. Chem. 29, 984 (1986). After coupling of the last amino acid, the peptide was cleaved from the resin with deprotection of the side chain protecting groups using anhydrous HF (20 mL) in the presence of anisole (2.0 mL) at 0°C for 30 min. After the evaporation of HF in vacuo, the residue was washed with anhydrous ether, the crude peptide was extracted with 0.2 M acetic acid, and the extract was diluted to 2 -L with deionized water. The pH of the aqueous solution was adjusted to 8-9 with conc. ammonium hydroxide. Nitrogen was bubbled through the solution to remove the ethyl mercaptan produced. The cyclization process took place within 24-48 hr. The reaction solution was lyophilized to yield a solid (320 mg). Chromatography (medium pressure reversed-phase column, 10% acetonitrile/H20-0.1% TFA) provided a partially purified product. Further purification using Sephadex® G-25 gel filtration (0.2M acetic acid) afforded the titled compound. MS(FAB) [M+H]⁺ 583; TLC R_{f} 0.22 (n-BuOH:HOAc:H₂O:EtOAc 1:1:1:1), R_{f} 0.48 (B:W:I:C, 65:20:15:3); HPLC k' 2.2 (Vydac 218 TP ODS column, 12% acetonitrile/H20-0.1% TFA, UV detection at 220 nm), k' 3.6 (Vydac 218 TP ODS column, gradient, A:acetonitrile, B:H₂O-0.1% TFA; 0-50% A during 10 min, UV detection at 220 nm); Peptide content 45%; Amino Acid Analysis: Asp(0.94), Gly(1.00), Arg(0.42), Cys(0.53).

### Example 2

### Preparation of N^{α}-acetyl-cyclo(S,S)-cysteinyl-arginyl-glycyl-aspartyl-(2-mercapto)phenylamide [cyclo-(S,S)-Ac-Cys-Arg-Gly-Asp-Man] (SEQ ID NO: 2)

### a) 2-(4-methylbenzyl)thioaniline (Man-4-MBzl)

To a solution of 2-thioaniline (5.0 mL, 42 mmol) in ethanol (50 mL), triethylamine (5.9 mL, 423 mmol) was added under argon. α-Bromo-p-xylene (7.78 g, 42 mmol) in ethanol (50 mL) was then added dropwise. The reaction mixture was stirred for 1 h, concentrated in vacuo to a small volume, diluted with anhydrous ether and filtered to remove triethyl amine hydrobromide. The filtrate was concentrated to dryness to give a yellow oil(5 g, 52%). Chromatography (silica gel, 20% ethyl acetate/hexane) yielded the titled compound as yellow oil (4.26 g).

### b) Fmoc-Asp(O-t-Bu)-Man(4-MBzl)

Fmoc-Asp(O-t-Bu) (5.0 g, 122 mmol) was dissolved in THF (50 mL), and N-methylmorpholine (1.3 mL, 118 mmol) was added, and the solution was cooled under argon in an ethanol/ice bath for 10 min. Isobutyl chloroformate (1.6 mL, 123 mmol) was added, the reaction stirred for 5 min, followed by the addition of a solution of Man(4-MBzl) (2.8 g, 122 mmol) in THF (50 mL). The cooled reaction mixture was stirred for 40 min and at room temperature for 4 h. The precipitated amine salt was filtered and the filtrate was evaporated to an oily material. The oil was dissolved in ethyl acetate (100 mL), washed with 1 M HCl (2 x 50 mL), saturated salt solution (1 x 50 mL), 10% sodium carbonate solution (1 x 50 mL) and saturated salt solution (1 x 50 mL). It was then dried (anhydrous Na2S04) and concentrated to an orange oil. Crystallization from methanol yielded the desired compound as a white solid (3.93 g, 26%). mp 129-130°C.H₂O

### c) Fmoc-Asp-Man(4-MBzl)

A mixture of Fmoc-Asp(O-tBu)-Man(4-MBzl) (3.5 g) and 50% TFA in methylene chloride (50 mL) was stirred at room temperature for 45 min. The solvent was evaporated and the product was precipitated by the addition of ether. The solid was collected and air dried to yield a white solid (2.23 g, 70%). mp 155-156°C

### d) Fmoc-Asp(O-Bzl-resin)-Man(4-MBzl)

To a swelled and washed hydroxymethyl resin (1.0 g, 1-mmol, CH₂Cl₂) was added a solution of Fmoc-Asp-Man(4-MBzl) (1.42 g, 2.5 mmol) and DCC (10 mL, 0.3 M in CH₂Cl₂) in dioxane (25 mL). The reaction was stirred for 18 h and washed sequentially with CH₂Cl₂, 1:1 mixture of CH₂Cl₂:EtOH and CH₂Cl₂. The unreacted hydroxymethyl resin was capped using benzoyl chloride (0.5 mL) in CH₂Cl₂ for 30 min. The resin was washed sequentially as above and dried to yield the resin-bound peptide (2.16 g).

### e) N^{α}-acetyl-cyclo(S,S)-Cys-Arg-Gly-Asp-Man (SEQ ID NO: 2)

The protected peptide-resin intermediate,Na-Ac-Cys(SEt)-Arg(Tos)-Gly-Asp(O-Bzl-resin) -Man(4-MBzl), was prepared from Asp(O-Bzl-resin)-Man(4-MBzl) using the method of Example 1(b), by sequentially coupling Boc-Gly, Boc-Arg(Tos) and Boc-Cys(SEt). After coupling of the last amino acid, the terminal Boc group was removed with TFA, and the peptide was acetylated using a mixture of acetic anhydride (10 eq.) and diisopropylethylamine (10 eq.) in dimethylformamide. The peptide was cleaved from the resin, cyclized and isolated as in Example 1(b), toprovide the crude peptide (310 mg). Chromatography (medium pressure ODS reversed-phase column, 10% acetonitrile:H₂O-0.1% TFA) affords the titled peptide (24 mg). MS(FAB) [M+H]⁺ 597.2; TLC R_{f} 0.57 (n-BUOH:HOAC:H₂O: EtOAc 1:1:1:1), R_{f} 0.23, (n-BuOH:H₂O:i-ProH:CA 65:20:15:3); HPLC k'= 4.9 (Vydac 218 Tp ODS column, 10% acetonitrile/H₂O-0.1% TFA, UV detection at 220 nm), k' 4.5 (Vydac 218 Tp ODS column, gradient, A:acetonitrile, B:H₂O-0.1% TFA, 0-50% A during 15 min, UV detection at 220 nm); Peptide content 44.6%; Amino Acid Analysis: Asp(1.00), Gly(1.03), Arg(0.94), Cys(0.5).

### Example 3

### Preparation of cyclo(S,S)-(2-mercapto)benzoyl-(N^{α}-methyl) arginyl-glycyl-aspartyl-(2-mercapto)phenylamide [cyclo-(S,S)-Mba-MeArg-Gly-Asp-Man]

The protected peptide-resin intermediate, Mba(SEt)-MeArg(Tos)-Gly-Asp(O- Bzl-resin)-Man(4-MBzl), was prepared, cleaved and cyclized in the same manner as Example 2 on 1.0 mmol scale. After the cyclization was completed, the aqueous solution was passed through a column of Amberlite® XAD-2 (1:1 acetonitrile:H₂O-0.1% TFA), concentrated and lyophilized to afford a crude peptide (100 mg). Chromatography (medium pressure ODS reversed-phase column, 30% acetonitrile/H₂O-0.1% TFA) yielded the titled compound (14 mg). MS(FAB) m/e [M+H]⁺ 602.3; TLC: R_{f} 0.63, (n-BuOH:HOAc:H₂O:EtOAC 1:1:1:1); HPLC k' 3.2 (Vydac 218 TP ODS column, 20% acetonitrile/H₂O-0.1% TFA, detection at 220 nm), k' 2.3, (Vydac 218 TP ODS column, gradient, A:acetonitrile, B:H₂O-0.1% TFA, 20-50% A during 10 min, detection at 220 nm); Peptide content 79% Amino Acid; Analysis: Asp(1.00), Gly(1.21)

### Example 4

### Preparation of cyclo-(S,S)-(2-mercapto)benzoyl-(N^{α}-methyl) arginyl-glycyl-aspartyl-(2-mercapto)phenylamide [cyclo(S,S)-Mba-MeArg-Gly-Asp-Man]

### a) Boc-Asp(O-cHex)-Man(4-MBzl)

To a cold solution of Boc-Asp(O-cHex), (31.5 g, 100 mmol) in THF (500 mL) and N-methylmorpholine (13.1 g, 120 mmol), isobutylchloroformate (15.6 mL, 1.2 mmol) was added dropwise. The reaction mixture was stirred for a few minutes and a solution of Man(4-MBzl) (22.0 g, 96 mmol) in THF (500 mL) was added. The reaction mixture was allowed to warm to room temperature and stirred for 18 h. Upon completion of the reaction, the reaction mixture was filtered and the filtrate was concentrated to dryness. The residue was dissolved in ethyl acetate (500 mL), and washed successively with 5% aqueous citric acid (3 x 150 mL), water (1 x 400 mL), 10% aqueous NaHCO₃ (1 x 400 mL), water (1 x 400 mL) and saturated salt solution (1 x 300 mL). The solution was dried (anhydrous K₂CO₃), filtered and concentrated to yield the titled compound (53 g).

### b) Asp(O-cHex)-Man(4-MBzl)

Boc-Asp(O-cHex)-Man(4-MBzl) (52 g) was treated with 50% TFA/methylene chloride (400 mL) for 45 min at room temperature. The solvent was evaporated and chased several times with methylene chloride to eliminate traces of TFA. The product precipitated as its TFA salt upon addition of ether. The solid was collected and air dried to yield a white solid (46.7 g, 88%).

### c) Boc-Gly-Asp(O-cHex)-Man(4-MBzl)

To a cold solution of Asp(O-cHex)-Man(4-MBzl) (46.7 g, 86.4 mmol) in DMF( 100 mL) diisopropylethylamine (15 mL, 86.1 mmol) was added. N-Hydroxybezotriazole (14.0 g, 104 mmol) was added followed by Boc-Gly (16.6 g, 94.8 mmol). The reaction mixture was stirred in the cold for a few minutes, and N-ethyl-N'-(dimethylaminopropyl)carbodiimide (18.2 g, 94.9 mmol) was added. The reaction mixture was allowed to warm to room temperature and stirred for 18 h. The reaction mixture was concentrated to a small volume and poured into 1.5 L of aqueous 10% K₂CO₃ . The precipitated product was collected by filtration and was washed with water to a neutral pH to afford the titled compound (50.6 g).

### d) Gly-Asp(O-cHex)-Man(4-MBzl)

The compound of Example 4(c) (11.7 g, 20 mmol) was treated with 50% TFA/CH₂Cl₂ (80 mL) as described in Example 4(b) to give 12.4 g of the titled compound.

### e) Boc-Me-Arg(Tos)-Gly-Asp(O-cHex)-Man(4-MBzl)

To an ice cold solution of the compound of Example 4(d) (12.4 g, 20 mmol) in DMF (20 mL), DIEA (3.6 mL, 20 mmol) was added. HOBt (3.4 g, 02 mmol) was added followed by Boc-N-MeArg(Tos) (10.1 g, 22 mmol). The reaction mixture was stirred for several min and EDC (4.4 g, 22 mmol) was added portionwise. The reaction mixture was allowed to warm to room temperature and stirred for 18 h. The reaction was concentrated to a small volume and poured into 10% aqueous K₂CO₃. The resulting solid was collected by filtration and washed with water to neutral pH to provide the titled compound (20.4 g).

### f) MeArg(Tos)-Gly-Asp(O-cHex)-Man(4-MBzl)

The compound of Example 4(e) (17.7 g, 19.5 mmol) was treated with 50% TFA/CH₂Cl₂ (80 mL) as in Example 4(b) to provide the TFA salt of the titled compound.

### g) Mba(SEt)-MeArg(Tos)-Gly-Asp(O-cHex)-Man(4-MBzl)

To a cold solution of the compound of Example 4(f) (20 mmol) in DMF (20 mL) was added DIEA (3.5 mL, 22 mmol) dropwise. Mba(SEt) (4.6 g, 22 mmol), EDC (4.2 g, 22 mmol) were added successively, followed by 4-N,N-dimethylaminopyridine (DMAP) (2.9 g, 24 mmol). The reaction mixture was allowed to warm to room temperature and stirring was continued for another 24 h. Another portion of Mba(SEt) (4.6 g, 22 mmol), DMAP (2.9 g,24 mmol) and EDC (4.2 g, 22 mmol) were added and stirring was continued for another 24 h to obtain complete reaction. The reaction mixture was concentrated, the residue was dissolved in CH₂Cl₂, the solution was washed successively with water, 5% aq. citric acid, water and saturated salt solution. The organic extracts was dried (anhydrous Na₂SO₄), filtered and concentrated to a solid residue (19.1 g). Chromatography (silica gel, 10% MeOH/CH₂Cl₂) affords the titled compound (9.0 g).

### h) cyclo-(S,S)-Mba-MeArg-Gly-Asp-Man

The protected linear peptide of Example 4(g) (8.5 g, 8.5 mmol), was treated with anhydrous HF (90 mL) and anisole (8.5 mL) at 0°C for 1 hr. The HF was removed at 0°C under vacuum, and the residue was washed with ether to yield a tan solid (5.0 g). The solid was dissolved in water (16 L), and the pH was adjusted to 8.0 using ammonium hydroxide. Nitrogen was bubbled through the solution to remove the ethyl mercaptan generated. After 7 days, the aqueous solution was passed through a column of Amberlite® XAD-2 (50% methanol/H₂O) to give 3.0 g after lyophilization. Further purification by flash chromatography (medium pressure ODS reversed-phase column, 21% acetonitrile/H₂O-0.1% TFA) gave 2.3 g of partially purified material. Final purification using Sephadex® G-15 gel filtration (0.2 M acetic acid) provided the titled compound (1.0 g).

### Example 5

### Preparation of cyclo(S,S)-(2-mercapto)benzoyl-(N^{α}-methyl)arginyl-glycyl-aspartyl-(3-phenyl)cysteineamide [cyclo-(S,S)-Mba-MeArg-Gly-Asp-Pcs-NH₂] (SEQ ID NO: 3)

Diasteromeric 3-phenylcysteine is prepared according to the method described by Nagai et al., in Peptide Chemistry, edited by,M. Ueki, Proceedings of the 26th Symposium on Peptide Chemistry, Tokyo, October 24-26, 1988, Protein Research Foundation, Minoh-shi, Osaka, pages 247-52. Using standard methods, this material is converted to S-(4-methoxyphenyl)-N-(t-butoxycarbonyl)-3-phenylcysteine. The protected peptide-resin intermediate, Mba(SEt)-Arg(Tos)-Gly-Asp(O-Bzl)-Pcs(4-MBzl)-MBHA, is synthesized by the solid-phase method on 4-methylbenzhydrylamine resin. All of the amino acids are protected by t-butyloxycarbonyl on the amino group, and are coupled sequentially using N,N-dicyclohexylcarbodiimide/1-hydroxybenzotriazole (DCC/HOBt) as set forth in Example 1. After coupling of the last amino acid, the peptide is cleaved from the resin with deprotection of the side chain protecting groups using anhydrous HF (20 mL) in the presence of anisole (2.0 mL) at 0°C for 30 min. After the evaporation of HF in vacuo, the residue is washed with anhydrous ether, the crude peptide is extracted with 0.2 M acetic acid, and the extract is diluted to 2 L with deionized water. The pH of the aqueous solution is adjusted to 7-8 with concentrated ammonium hydroxide and Nitrogen is bubbled through the solution. After 24-48 hr. The reaction solution is lyophilized to yield a crude product. Chromatography (medium pressure reversed-phase column, acetonitrile/H₂O-0.1% TFA) provides the titled product.

### Example 6

### Preparation of cyclo-(S,S)-Mba-Sar-Arg-Gly-Asp-Man (SEQ ID NO: 4)

### a) Boc-Asp(Oc-Hex)-Man(4-MBzl)

To a cold solution of Boc-Asp(O-cHex) (31.5 g, 100 mmol) and N-methylmorpholine (13.1 g, 120 mmol) in THF (500 mL), isobutylchloroformate (15.6 mL, 1.2 mmol) was added dropwise. The reaction mixture was stirred for a few minutes, then a solution of Man(4-MBzl) (22.0 g, 96 mmol) in THF (500 mL) was added. The reaction mixture was allowed to warm to room temperature and stirred for 18 h. Upon completion of the reaction (monitored by TLC), the amine salt was filtered off and the filterate was concentrated to dryness. The residue was dissolved in ethyl acetate (500 mL) and washed successively with 5% aqueous citric acid (3x150 mL), water (1x400 mL), aqueous NaHCO₃ (1x300 mL), water (1x400 mL) and saturated salt solution (1x30 mL). The organic solvent was dried (anhydrous K₂CO₃), filtered and concentrated to yield the title product (53 g).

### b) Asp(O-cHex)-Man(4-MBzl)

Boc-Asp(O-cHex)-Man(4-MBzl), (1) (52 g) was treated with 50% TFA solution in methylene chloride (400 mL) for 45 min at room temperature. The solvent was removed and the residue was azeotroped several times from methylene chloride to eliminate traces of TFA, and the product was precipitated by the addition of ether. The solid was collected and air dried to yield a white solid (46.7 g,88%).

### c) Boc-Gly-Asp(O-cHex)-Man(4-MBzl)

To a cold solution of the compound of Example 6b (46.7 g, 86.4 mmol) in DMF (100 mL), DIEA (15 mL, 86.1 mmol) was added to bring the pH to neutrality. HOBt (14.0 g, 104 mmol) was added followed by Boc-Gly (16.6 g, 94.8 mmol). The reaction was stirred in the cold for a few min, then EDC (18.2 g, 94.9 mmol) was added portionwise. The reaction mixture was allowed to warm to room temperature and stirred for 18 h. The reaction mixture was concentrated to a small volume and poured into 1.5 L of aqueous 10% K₂CO₃. The precipitated product was collected by filtration and washed with water to yield the titled compound (50.6 g).

### d) Gly-Asp(O-cHex)-Man(4-MBzl)

The compound of Example 6c (2.92 g, 5 mmol ) was treated with 50% TFA (80 mL) according to the procedure of Example 6b to yield the titled compound (2.93 g , 98%).

### e) Boc-Arg(Tos)-Gly-Asp(O-cHex)-Man(4-MBzl)

To an ice cold solution of the compound of Example 6d (1.4 g,2.3 mmol) in DMF (4 mL), DIEA (410 µL, 2.3 mmol) was added to bring the pH to neutrality. HOBt (380 mg, 2.76 mmol) was added followed by Boc-Arg(Tos) (1.2 g, 1.32 mmol). The reaction mixture was stirred for a few min, then EDC (493 mg, 1.32 mmol) was added. The reaction mixture was allowed to warm to room temperature and stirred for 18 h. The reaction mixture was concentrated to dryness, and the residue was dissolved in ethyl acetate and washed successively with water (1x), 10% K₂CO₃ (2x), water (1x) and brine (1x). The ethyl acetate extract was dried (anhydrous K₂CO₃) filtered and concentrated to yield the titled compound (1.91 g).

### f) Arg(Tos)-Gly-Asp(O-cHex)-Man(4-MBzl)

The compound of Example 6e (1.9 g, 2.1 mmol) was treated with 50% TFA (10 mL) according to the procedure of Example 6b to yield the titled compound (1.59 g).

### g) Boc-Sar-Arg(Tos)-Gly-Asp(O-cHex)-Man(4-MBzl)

To an ice cold solution of the compound of Example 6f (0.8 g, 2.3 mmol) in DMF (2 mL), DIEA (153 µL) was added to bring the pH to neutrality. HOBt (143 mg, 2.76 mmol) was added followed by Boc-Sar (183 mg, 2.53 mmol). The reaction mixture was stirred for a few min, then EDC (186 mg, 2.53.mmol) was added portionwise. The reaction mixture was allowed to warm to room temperature and stirred for 18 h. It was then concentrated to dryness, and the residue was dissolved in ethyl acetate and washed successively with water (1x), 10% K₂CO₃ (2x), water (1x) and brine (1x). The ethyl acetate extract was dried (anhydrous K₂CO₃), filtered and concentrated to yield the titled compound (0.78 g).

### h) Sar-Arg(Tos)-Gly-Asp(O-cHex)-Man(4-MBzl)

The compound of Example 6g (780 m g, 0.8 mmol ) was treated with 50% TFA (5 mL) according to the procedure of Example 6b to yield the titled compound (500 mg).

### i) Mba(SEt)-Sar-Arg(Tos)-Gly-Asp(O-cHex)-Man(4-MBzl)

To an ice cold solution of the compound of Example 6h (0.85 g, 0.5 mmol) in DMF (2 mL), DIEA (89 µL) was added to bring the pH to neutrality. HOBt (248 mg, 1.8 mmol) was added followed by Mba(SEt) (394 mg, .55 mmol). The reaction mixture was stirred for a few min, then EDC ( 353 mg, .55 mmol) was added portionwise. The reaction mixture was allowed to warm to room temperature and stirred for 18 h. It was then concentrated to dryness and the residue was dissolved in ethyl acetate and washed successively with water (1x), 10% K₂CO₃ (2x), water (1x), 5% citric acid (2x), water (3x) and brine (1x). The ethyl acetate extract was dried (anhydrous Na₂SO₄), filtered and concentrated to yield the titled compound (0.45 g).

### j) cyclo (S,S)-Mba-Sar-Arg-Gly-Asp-Man (SEQ ID NO: 4)

The protected linear peptide of Example 6i (423 mg), was treated with anhydrous HF (10 mL) in the presence of anisole (1 mL) at 0°C for 1 h. The HF was removed at 0°C under vacuum, and the residue was triturated with ether . The solid was dried in vacuo to yield the crude cyclized peptide (235 mg). It was purified by gel filtration (Sephadex® G-15, 1% acetic acid/water) . The appropriate fractions were pooled and lyophilized to yield the semipurified titled compound (65 mg). An aliquot of the semipurified peptide (18 mg) was purified by preparative HPLC (5 µ Altex Ultrasphere® ODS, 10 mm x 25 cm, 20% acetonitrile/water-0.1% trifluoroacetic acid, UV detection at 220 nm to yield the purified title compound (6.0 mg). MS (FAB) m/e 659.1 [M+H]⁺; HPLC k' 6.6 (5 µ Altex Ultrasphere® ODS, 4.5 mm x 25 cm, gradient, A:acetonitrile B:water-0.1% trifluoroacetic acid, 10%-50% acetonitrile in 20 min, UV detection at 220 nm), k' 6.9 (Altex Ultrasphere® ODS, 20% acetonitrile/water-0.1% trifluoroacetic acid, UV detection at 220 nm), Amino Acid Analysis: Asp (1.00), Gly (1.03), Arg (1.11).

### Example 7

### Preparation of cyclo-(S,S)-Mba-Sar-MeArg-Gly-Asp-Man (SEQ ID NO: 5)

### a) Boc-MeArg(Tos)-Gly-Asp(O-cHex)-Man(4-MBzl)

To an ice cold solution of the compound of Example 6d (0.6 g, 1.0 mmol) in DMF (42 mL), DIEA (130 µL, 2.2 mmol) was added to bring the pH to neutrality. HOBt (165 mg, 1.2 mmol) was added followed by Boc-MeArg(Tos) (0.5 g, 1.1 mmol). The reaction mixture was stirred for a few min, then EDC (210 mg, 1.2 mmol) was added. The reaction mixture was allowed to warm to room temperature and stirred for 18 h. It was concentrated to dryness and the residue was dissolved in ethyl acetate and washed successively with water (1x), 10% K₂CO₃(2x), water (lx) , 5% citric acid (2x) and brine (lx). The ethyl acetate extract was dried (anhydrous Na₂SO₄), filtered and concentrated to yield the titled compound (780 mg, 86%).

### b) MeArg(Tos)-Gly-Asp(O-cHex)-Man(4-MBzl)

The compound of Example 7a (789 mg, 0.86 mmol ) was treated with 50% TFA (4 mL) according to the procedure of Example 6b to yield the titled compound.

### c) Boc-Sar-MeArg(Tos)-Gly-Asp(O-cHex)-Man(4-MBzl)

To an ice cold solution of the compound of Example 7b (0.8 g,0.86 mmol) in DMF (2 mL), DIEA (153 µL) was added to bring the pH to neutrality. HOBt (143 mg, 1.03 mmol) was added, followed by Boc-Sar (183 mg, .95 mmol). The reaction mixture was stirred for a few min and EDC (186 mg, .95 mmol) was added. The reaction mixture was allowed to warm to room temperature and stirred for 18 h. The reaction mixture was concentrated to dryness, and the residue was dissolved in ethyl acetate and washed successively with water (1x), 10% K₂CO₃(2x), water (1x), 1N HCl (1x), water (3x) and brine (1x). The ethyl acetate extract was dried (anhydrous Na₂SO₄), filtered and concentrated to yield the titled compound (0.65 g).

### d) Sar-MeArg(Tos)-Gly-Asp(O-cHex)-Man(4-MBzl)

The compound of Example 7c (650 mg, 0.8 mmol) was treated with 50% TFA (5mL) according to the procedure of Example 6b to yield the titled compound (510 mg).

### e) Mba(SEt)Sar-MeArg(Tos)-Gly-Asp(O-cHex)-Man(4-MBzl]

To an ice cold solution of the compound of Example 6d (0.5 g, 0.5 mmol) in DMF (2 mL), DIEA (120 mL, 0.6 mmol) was added to bring the pH to neutrality. HOBt (150 mg, 0.55 mmol) was added, followed by Mba (SEt) (394 mg, 0.55 mmol). The reaction mixture was stirred for a few min, then EDC (260 mg, 0.55 mmol) was added portionwise. The reaction mixture was allowed to warm to room temperature, stirred for 18 h and concentrated to dryness. The residue was dissolved in ethyl acetate and washed successively with water (1x), 10% K₂CO₃(2x), water (1x), 5% citric acid (2x), water (3x) and brine (1x). The ethyl acetate extract was dried (anhydrous Na₂SO₄), filtered and concentrated to yield the titled compound (0.46 g).

### f) cyclo-(S,S)-Mba-Sar-MeArg-Gly-Asp-Man (SEQ ID NO: 5)

The protected linear peptide of Example 7d (450 mg) was treated with anhydrous HF (10 mL) in the presence of anisole ( 1 mL) at 0°C for 1 h. The HF was removed at 0°C under vacuum, and the residue was triturated with ether . The solid was dried in vacuo to yield the crude cyclized peptide (268 mg). It was purified by gel filtration (Sephadex® G-15, 1% acetic acid/water). The appropriate fractions were pooled and lyophilized to yield the semipurified peptide. An aliquot of the semipurified peptide (65 mg) was further purified by HPLC (5 µ Altex Ultrasphere® ODS, 10 mm x 25 cm, 20% acetonitrile/water-0.1% trifluoroacetic acid, UV detection at 220 nm) to yield the titled compound (6.0 mg). MS (FAB) m/e 673.2 [M+H]⁺; TLC R_{f} 0.68 (n-BuOH:HOAc:H2O: EtOAc 1:1:1:1), R_{f} 0.74 ( n-BuOH:HOAc:H2O:pyridine 15:5:10:10); HPLC k' 11.8 (5 µ Altex Ultrasphere® ODS, gradient, A:acetonitrile B:water-0.1% trifluoroacetic acid, 10%-50% acetonitrile in 20 min, UV detection at 220 nm), k' 8.6 (Altex Ultrasphere® ODS, 20% acetonitrile/water-0.1% trifluoroacetic acid, UV detection at 220 nm); Amino Acid Analysis: Asp (1.04), Gly (1.00).

### Example 8

### Preparation of cyclo-(S,S)-Mba-Arg-Gly-Asp-Man

### a) Mba(SEt)-Arg(Tos)-Gly-Asp(O-cHex)-Man(4-MBzl)

To an ice cold solution of the compound of Example 6f (0.80 g, 0.88 mmol) in DMF (2 mL), DIEA (153 µL) was added to bring the pH to neutrality. Mba(SEt) (380 mg, .97 mmol) was added, the reaction mixture was stirred for a few min, and EDC (340 mg, .97 mmol) was added, followed by 4-dimethylaminopyridine (215 mg). The reaction mixture was allowed to warm to room temperature, stirred for 18 h, and concentrated to dryness. The residue was taken into ethyl acetate and washed successively with water (1x), 10% K₂CO₃(2x), water (1x), 5% citric acid (2x), water (3x) and brine (1x). The ethyl acetate extract was dried (anhydrous Na₂SO₄), filtered and concentrated to yield the titled compound (0.41 g).

### b) cyclo-(S,S)-Mba-Arg-Gly-Asp-Man

The protected linear peptide of Example 8b (223 mg) was treated with anhydrous HF (10 mL) in the presence of anisole (1 mL) at 0°c for 1 h. The HF was removed at 0°C under vacuum, and the residue was triturated with ether . The solid was dried in vacuo to yield the crude cyclized peptide (92 mg). An aliquot (20 mg) was purified by HPLC (5 µ Altex Ultrasphere® ODS, 10 mm x 25 cm, 18% acetonitrile/water-0.1% trifluoroacetic acid, UV detection at 220 nm) to yield the purified titled compound (5.0 mg). MS (FAB) m/e 588 [M+H]⁺; TLC R_{f} 0.68 (n-BuOH:HOAc:H₂O:EtOAc 1:1:1:1); and 0.70 ( n-BuOH:HOAc:H₂O:pyridine 15:5:10:10); HPLC k' 6 (5 µ Altex Ultrasphere® ODS, gradient, A:acetonitrile B:water-0.1% trifluoroacetic acid, 10%-50% acetonitrile in 20 min, UV detection at 220 nm), k' 3.6 (5 µ Altex Ultrasphere® ODS, 20% acetonitrile/water-0.1% trifluoroacetic acid, UV detection at 220 nm); Amino Acid Analysis: Asp (0.81), Gly (1.28), Arg (1.00).

### Example 9

### Preparation of cyclo-(S,S)-Mba-D-MeArg-Gly-Asp-Man

### a) Boc-D-MeArg(Tos)-Gly-Asp(O-cHex)-Man(4-MBzl)

To an ice cold solution of the compound of Example 7d (0.6 g, 1.0 mmol) in DMF (42 mL), DIEA (130 µL, 2.2 mmol) was added to bring the pH to neutrality. HOBt (202 mg, 1.5 mmol) was added, followed by Boc-D-MeArg(Tos) (0.663 g, 1.5 mmol). The reaction mixture was stirred for a few min, then EDC (288 mg, 1.5 mmol) was added. The reaction mixture was allowed to warm to room temperature, stirred for 18 h and concentrated to dryness. The residue was dissolved in ethyl acetate and washed successively with water (1x), 10% K₂CO₃(2x), water (1x), 5% citric acid (2x), water (3x) and brine (1x). The ethyl acetate extract was dried (anhydrous Na₂SO₄), filtered and concentrated to yield the titled compound (500 mg).

### b) D-MeArg(Tos)-Gly-Asp(O-cHex)-Man(4-MBzl)

The compound of Example 9a (500 mg, 0.5 mmol ) was treated with 50% TFA (5 mL) according to the procedure of Example 6b to yield the titled compound (658 mg).

### c) Mba(SEt)-D-MeArg(Tos)-Gly-Asp(O-cHex)-Man(4-MBzl)

To an ice cold solution of the compound of Example 9b (658 mg, 0.71 mmol) in DMF (4 mL), DIEA (120 mL, 0.85 mmol) was added to bring the pH to neutrality. Mba (SEt) (306 mg, 1.42 mmol) was added, the reaction mixture was stirred for a few min, and EDC (274 mg, 1.42 mmol) and 4-dimethylaminopyridine (174 mg, 1.42 mmol) were added. The reaction mixture was allowed to warm to room temperature, stirred for 18 h, and concentrated to dryness. The residue was dissolved in ethyl acetate and washed successively with water (1x), 10% K₂CO₃(2x), water (1x), 5% citric acid (2x), water (3x) and brine (1x). The ethyl acetate extract was dried(anhydrous Na₂SO₄), filtered and concentrated to yield the titled compound (0.37 g).

### d) cyclo-(S,S)-Mba-D-MeArg-Gly-Asp-Man

The protected linear peptide of Example 9c (370 mg), was treated with anhydrous HF (10 mL) in the presence of anisole ( 1 mL) at 0°C for 1 h. The HF was removed at 0°C under vacuum, and the residue was triturated with ether. The solid was dried in vacuo to yield the crude cyclized peptide (246 mg). It was purified by gel filtration (Sephadex® G-15, 1% acetic acid/water) . The appropriate fractions were pooled and lyophilized to yield the purified titled compound (13 mg). MS (FAB) m/e 602 [M+H]⁺; TLC R_{f} 0.74 (n-BuOH:HOAc:H₂O:EtOAc 1:1:1:1), R_{f} 0.68 ( n-BuOH:HOAc:H₂O:pyridine 15: 5:10:10); HPLC k' 6.7 (5 µ Altex Ultrasphere® ODS, gradient, A:acetonitrile B:water-0.1% trifluoroacetic acid, 10%-50% acetonitrile in 20 min, UV detection at 220 nm), k' 6.1 (5 µ Altex Ultrasphere® ODS, 21% acetonitrile/ water-0.1% trifluoroacetic acid, UV detection at 220 nm); Amino Acid Analysis: Asp (1.00), Gly (1.21).

### Example 11

### Preparation of N^{α}Ac-cyclo-(S,S)-Cys-MeArg-Gly-Asp-(2R,3S)Pcs-NH₂ (SEQ ID NO: 6)

The protected pentapeptide resin Boc-Cys(SEt)-MeArg(Tos)-Gly-Asp(O-cHex)-(2R,3S)-3-phenylcysteine (4MBzl)-MeBHA was prepared according to Example 1 on a 0.5 mmol scale. After removal of the N-terminal Boc group with 50% TFA in methylene chloride and neutralizing the resulting TFA salt with 7% DIEA in methylene chloride, the resin-bound peptide was acetylated with acetic anhydride (0.47 mL), and DIEA (0.86 mL) in methylene chloride (20 mL) for 40 min.

The peptide was cleaved from the resin with removal of the side chain protecting groups by treatment with anhydrous liquid HF (10 mL) in the presence of anisole (1 mL) at 0°C for 50 min. After removal of the HF under vacuum, the resin was washed with ethyl ether and air-dried. The resin was then extracted with 1% acetic acid/water (2 x 30 mL) followed by 10% acetic acid/water (2 x 30 mL). The combined extracts were diluted to 1 L with deionized water. The pH of the aqueous solution was adjusted to 7.65 with con. ammonium hydroxide, and cyclized by bubbling an inert gas such as argon in which the free sulfhydryl group, generated from the cleavage of the MBzl protecting one of sulfure, displaces the SEt group protecting the other sulfur. The cyclization process was achieved in 48-72 h. The solution was chromatographed (ODS silica, step gradient: a) water b) 12% acetonitrile-0.1% TFA-water). The appropriate fractions were pooled, evaporated to dryness and the residue was lyophilized from 1% acetic acid/water to yield the purified titled peptide (80 mg). MS(FAB)m/e 682.2 [M+H]⁺; TLC R_{f} 0.56 (n-BuOH:HOAc:H₂O: EtOAc 1:1:1:1), R_{f} 0.61 (n-BuOH:HOAc:H₂O:pyridine 15:5:10:10); HPLC k' 13.1 (5 µ 5µ Altex Ultrasphere® ODS, gradient, A:acetonitrile B:water-0.1% trifluoroacetic acid, 0-50% acetonitrile in 20 min, UV detection at 220 nm), k' 5.7 (5 µ 5µ Altex Ultrasphere® ODS, 15% acetonitrile/water-0.1% trifluoroacetic acid, UV detection at 220 nm), Amino Acid Analysis: Asp(0.97), Gly(1.00), Cys(0.38), β-phenyl-Cys(0.63).

### Example 12

### Preparation of N^{α}Ac-cyclo-(S,S)-Cys-MeArg-Gly-Asp-(2R,3R)Pcs-NH₂ (SEQ ID NO: 7)

The protected pentapeptide resin Boc-Cys(SEt)-MeArg(Tos)-Gly-Asp(O-cHex)-(2R,3R)-3-phenylcysteine (4MBzl)-MeBHA was prepared according to Example 1 on a 0.5 mmol scale. After removal of the N-terminal Boc group with 50% TFA in methylene chloride and neutralizing the resulting TFA salt with 7% DIEA in methylene chloride, the resin-bound peptide was acetylated with acetic anhydride (0.47 mL) and DIEA (0.86 mL) in methylene chloride (20 mL) for 40 min.

The peptide was cleaved from the resin with removal of the side chain protecting groups by treatment with anhydrous HF (10 mL) in the presence of anisole (1 mL) at 0°C for 50 min. After removal of the HF under vacuum, the resin was washed with ethyl ether and air-dried. The resin was then extracted with 1% acetic acid/water (2 x 30 mL) followed by 10% acetic acid/water (2 x 30 mL). The combined extracts were diluted to 1 L with deionized water. The pH of the aqueous solution was adjusted to 7.65 with ammonium hydroxide, and cyclized by bubbling argon through the solution (argon drives off ethanethiol liberated by free sulfhydryl group of Pcs nucleophically displacing the SEt group protecting cysteine sulfhydryl). After 72 h, the solution was chromatographed (ODS silica, step gradient: a) water b) 11% acetonitrile/water-0.1% TFA). The appropriate fractions were pooled, evaporated to dryness and the residue. was lyophilized from 1% acetic acid/water to yield titled peptide (92 mg). MS(FAB) m/e 682 [M+H]⁺; TLC R_{f} 0.70 (n-BuOH:HOAc:H₂O:EtOAc 1:1:1:1), 0.67 (n-BuOH:HOAc:H₂O:pyridine 15:5:10:10); HPLC k' 8.35 (5µ Altex Ultrasphere® ODS, gradient, A:acetonitrile B:water-0.1% trifluoroacetic acid, 0-50% acetonitrile in 20 min, UV detection at 220 nm), k' 4.45 (5µ Altex Ultrasphere® ODS, 12% acetonitrile/water-0.1% trifluoroacetic acid, UV detection at 220 nm); Amino Acid Analysis: Asp(1.00), Gly(0.96), Cys(0.38), β-phenylCys(0.56).

### Example 15

### Preparation of N^{α}Ac-cyclo-(S,S) Cys-Arg-Gly-Asp-Ser-MeArg-Gly-Asp-Ser-Cys-NH₂ (SEQ ID NO: 10)

The protected decapeptide resin Boc-Cys(SEt)-Arg(Tos)-Gly-Asp(O-cHex)-Ser(Bzl)-MeArg(Tos)-Gly-Asp(O-cHex)-Ser(Bzl)-Cys(4MBzl)-MeBHA was prepared according to Example 1 on a 1.0 mmol scale. After removal of the N-terminal Boc group with 50% TFA in methylene chloride and neutralizing the resulting TFA salt with 7% DIEA in methylene chloride, the resin-bound peptide was acetylated with acetic anhydride (0.94 mL), and DIEA (1.72 mL) in DMF (30 mL) for 40 min.

The peptide was cleaved from the resin with removal of the side chain protecting groups by treatment with anhydrous liquid HF (30 mL) in the presence of anisole (3 mL) at 0°C for 50 min. After removal of the HF under vacuum, the resin was washed with ethyl ether and air-dried. The resin was then extracted with 1% acetic acid/water (2 x 30 mL) followed by 10% acetic acid/water (2 x 30 mL). The combined extracts were diluted to 1.5 L with deionized water. The pH of the aqueous solution was adjusted to 7.9 with ammonium hydroxide and argon was bubbled through the reaction mixture for 72 h. The solution was lyophilized to yield a crude peptide (409 mg). An aliquot (209 mg) was purified by gel filtration (Sephadex® G-15, 1% acetic acid/water). The appropriate fractions were pooled and lyophilized to yield the purified titled compound (20 mg). MS(FAB) m/e 1108.3 [M+H]⁺; TLC R_{f} 0.23 (n-BuOH:HOAc:H2O:EtOAc 1:1:1:1), R_{f} 0.45 (n-BuOH:HOAc:H20:pyridine 15:5:10:10); HPLC k' 5.6 (5µ Altex Ultrasphere® ODS, gradient, A:acetonitrile B:water-0.1% trifluoroacetic acid, 0-50% acetonitrile in 20 min, UV detection at 220 nm), k' 0.6 (5µ Altex Ultrasphere® ODS, 10% acetonitrile/water-0.1% trifluoroacetic acid, UV detection at 220 nm); Amino Acid Analysis: Asp(2.11), Gly(2.06), Cys(1.84), Ser(2.00), Arg(0.84).

### Example 16

### Preparation of N^{α}Ac-cyclo-(S,S)-Cys-MeArg-Gly-Asp-Ser-Arg-Gly-Asp-Ser-Cys-NH₂ (SEQ ID NO: 11)

The protected decapeptide resin Boc-Cys(SEt)-MeArg(Tos)-Gly-Asp(O-cHex)-Ser(Bzl)-Arg(Tos)-Gly-Asp(O-cHex)-Ser(Bzl)-Cys(4MBzl)-MeBHA was prepared according to Example 1 on a 1.0 mmol scale. After removal of the N-terminal Boc group with 50% TFA in methylene chloride and neutralizing the resulting TFA salt with 7% DIEA in methylene chloride, the resin-bound peptide was acetylated with acetic anhydride (0.94 mL) and DIEA (1.72 mL) in DMF (30 mL) for 40 min.

The peptide was cleaved from the resin with removal of the side chain protecting groups by treatment with anhydrous liquid HF (10 mL) in the presence of anisole (1 mL) at 0°C for 50 min. After removal of the HF under vacuum, the resin was washed with ethyl ether and air-dried. The resin was then extracted with 1% acetic acid/water (2 x 30 mL) followed by 10% acetic acid/water (2 x 30 mL). The combined extracts were diluted to 1.5 L with deionized water. The pH of the aqueous solution was adjusted to 7.6 with ammonium hydroxide and argon was bubbled through the reaction mixture for 72 h. The solution was chromatographed (ODS silica, step gradient: a) water b) 5% acetonitrile-0.1% TFA-water). The appropriate fractions were pooled, evaporated to dryness and the residue was lyophilized from 1% acetic acid/water to yield a semipurified peptide (800 mg). An aliquot (200 mg) was purified by gel filtration (Sephadex® G-15, 1% acetic acid/water). The appropriate fractions were pooled and lyophilized to yield the purified titled compound (90 mg). MS(FAB) m/e 1108.4 [M+H]⁺; TLC R_{f} 0.29 (n-BuOH:HOAc:H₂O:EtOAc 1:1:1:1), R_{f} 0.32 (n-BuOH:HOAc:H₂O:pyridine 15:5:10:10); HPLC k' 6.6 (5µ Altex Ultrasphere® ODS, gradient, A:acetonitrile B:water-0.1% trifluoroacetic acid, 5 -50% acetonitrile in 20 min, UV detection at 220 nm), k' 6.6 (5µ Altex Ultrasphere® ODS, 4% acetonitrile/water-0.1% trifluoroacetic acid, UV detection at 220 nm); Amino Acid Analysis: Asp(2.00), Gly(2.21), Cys(1.45), Ser(1.70), Arg(1.12).

### Example 19

### Preparation of cyclo(1^{α},6^{γ})-Gly-MeArg-Gly-Asp-Ser-Glu-NH₂ (SEQ ID NO:

The protected hexapeptide resin Boc-Gly-MeArg(Tos)-Gly-Asp(Bzl)-Ser(Bzl)Glu(t-But)-BHA was prepared according to Example 1 on a 1 mmol scale. The N-terminal Boc group on glycine and the t-butyl ester on the glutamic acid sidechain were removed with 50% TFA in methylene chloride. The resulting TFA salt was neutralized with 7% DIEA in methylene chloride and the resin-bound peptide was cyclized between the alpha amine of glycine and the gamma carboxy group of glutamic acid using the BOP reagent (3 mmol) and DIEA (6 mmol) in DMF.

The peptide was cleaved from the resin with removal of the side chain protecting groups by treatment with anhydrous liquid HF (30 mL) in the presence of anisole (3 mL) at 0°C for 50 min. After removal of the HF under vacuum, the resin was washed with ethyl ether and air-dried. The resin was then extracted with 1% acetic acid/water (2 x 30 mL) followed by 10% acetic acid/water (2 x 30 mL). The combined extracts were lyophilized to yield a crude peptide (680 mg, 100%).

The crude peptide was purified by flash chromatgraphy (ODS reversed-phase silica, 1.5% acetonitrile/water-0.1% TFA). The appropriate fractions were pooled, evaporated to dryness and the residue was lyophilized from 1% acetic acid/water to yield a partially purified peptide (356.8 mg). An aliquot of partially purified peptide (66 mg) was further purified by HPLC (5µ Altex Ultrasphere® ODS, 2% acetonitrile/water-0.1% trifluoroacetic acid, UV detection at 220 nm) to yield the purified titled compound (31 mg). MS(FAB) m/e 615 [M+H]⁺; TLC R_{f} 0.36 TLC R_{f} 0.38 (n-BuOH:HOAc:H₂O:EtOAc 1:1:1:1), R_{f} 0.42 (n-BuOH:HOAc:H₂O:pyridine 15:5:10:10); HPLC k' 7.43 (5µ Altex Ultrasphere® ODS, gradient, A:acetonitrile B:water-0.1% trifluoroacetic acid, 0-50% acetonitrile in 20 min, UV detection at 220 nm), k' 4.43 (5µ Altex Ultrasphere® ODS, 3% acetonitrile/water-0.1% trifluoroacetic acid, UV detection at 220 nm); Amino Acid Analysis: Asp(1.00),Gly (1.88), Ser(0.85), Glu(1.05).

### Example 20

### General procedure for solid phase peptide synthesis of Homodetic peptides - FMOC-SASRIN Method

Cyclic homodetic peptides were synthesized by solid phase peptide synthesis using SASRIN resin (Bachem) as the support. Protected amino acids were added sequentially starting from the carboxy terminus until the desired sequence was obtained. The 9-fluorenylmethoxycarbonyl (FMOC) group was used for protection of the alpha amino group. Side chain functional groups were protected as follows: arginine and N^{α}-methylarginine, tosyl (Tos) or 4-methoxyl-2,3,6-trimethylphenylsulfonyl (Mtr); serine, benzyl ether (Bzl); glutamic and aspartic, benzyl (Bzl) or t-butyl ester (t-Bu). Removal of the FMOC group was accomplished by treatment with 20% piperidine in DMF. Amino acids were coupled to the growing peptide using 2-3 equivalents of the FMOC-protected amino acid, 2-3 equivalents of HOBt in DMF and 2-3 equivalents of DCC in methylene chloride. Completeness of coupling was checked by the ninhydrin test and couplings were repeated as necessary. The general protocol is given below.

| | | |
|---|---|---|
| 1. | Wash with DMF | 5x1 min. |
| 2. | Wash with 20% piperidine in DMF | 1x1 min |
| 3. | Deprotect with 20% pip.in DMF | 1x7 min |
| 4. | Wash with DMF | 10x1 min |
| 5. | Test for deprotection by ninhydrin | |
| 6. | FMOC-AA + HOBt in DMF, do not drain | 2 min |
| 7. | DCC in methylene chloride | 2 h |
| 8. | Wash with DMF | 5x1 min |
| 9. | Wash with methylene chloride | 3x1 min |
| 10. | Test for complete coupling by ninhydrin | |
| 11. | Continue from step 1 if negative ninhydrin obtained or recouple from step 6 if positive ninhydrin obtained. | |

Upon completion of the desired protected peptide, the FMOC protection of the N-terminus is removed, and the protected peptide is cleaved from the resin using 1-2% trifluoroacetic acid (TFA) in methylene chloride (3x15 min). The methylene chloride/TFA extracts are combined and concentrated to yield the protected peptide.

To effect cyclization, the protected peptide (0.2 mmol, 0.4 mM solution in dry DMF) is treated with N-methylmorpholine (5 equivalents) and 1-propanephosphonic acid cyclic anhydride (PPA) (1.2 equivalents) at 0°C for 1 h. Another equivalent of PPA is added, and the reaction mixture is allowed to stir at room temperature for 18 h. The solvent is removed, and the residue is triturated with water to yield the desired cyclic protected peptide. Protected sidechain functional groups are deprotected by treatment with anhydrous HF in the presence of anisole (10%) at 0°C for 50 min. The HF is removed under vacuum, the crude peptide is dissolved in acetic acid (0.2M), washed with ether (3X) and lyophilized to yield the desired crude peptide.

### Example 21

### Preparation of cyclo-(1,8)-MeArg-Gly-Asp-Phe -Arg-Gly-Asp-Phe (SEQ ID NO: 16)

The protected octapeptide resin FMOC-Asp(O-t-Bu)-Phe-MeArg(Tos)-Gly-Asp(O-t-Bu)-Phe-Arg(Mtr)-Gly-SASRIN was prepared according to Example 20 on 1 mmol·scale. After the removal of the N-terminal FMOC group with 20% piperidine in DMF, the resin-bound peptide was cleaved from the resin with 1% TFA in methylene chloride to give the protected linear peptide (1.22 g, 84%) as a white solid. The protected peptide (585 mg, 0.4 mmol) in DMF (1 L ) was treated with NMM (220 µL, 2.0 mmol) and PPA (304 µL,0.48 mmol) at 0°C. After an hour stirring at 0°C, another portion of PPA was added and stirring was continued for 18 h. Removal of the solvent and triturating the residue with water afforded the cyclized protected peptide cyclo-(1,8)-MeArg(Tos)-Gly-Asp(O-t-Bu)-Phe-Arg(Mtr)-Gly-Asp(O-t-Bu)-Phe (550 mg, 95%). The peptide was treated with anhydrous HF (30 mL) in the presence of anisole (3 mL) at 0°C for 50 min. After removal of HF under vacuum, the peptide was dissolved in 60 mL of 0.2 M acetic acid, washed with ether (3 x 20 mL) and lyophilized to give the crude cyclo-(1,8)-MeArg-Gly-Asp-Phe-Arg-Gly-Asp-Phe (406 mg). The crude peptide (200 mg) was purified by gel filtration (Sephadex® G-15, 1% acetic acid/water). The appropriate fractions were pooled and lyophilized. The peptide was further purified by HPLC (5µ Altex Ultrasphere® ODS, 5 µL 10 mm x 25 cm, gradient, A:acetonitrile B:water-0.1% trifluoroacetic acid, 10-50% acetonitrile in 10 min, UV detection at 220 nm) to yield the purified titled compound (26 mg). MS(FAB) m/e 965.4 [M+H]⁺; TLC R_{f} 0.65 (n-BuOH:HOAc:H₂O:EtOAc 1:1:1:1), R_{f} 0.63 (n-BuOH:HOAc:H₂O:pyridine 15:5:10:10); HPLC k' 8.14 (5µ Altex Ultrasphere® ODS, gradient, A:acetonitrile B:water-0.1% trifluoroacetic acid, 0-50% acetonitrile in 20 min, UV detection at 220 nm), k' 9 (5µ Altex Ultrasphere® ODS, 14% acetonitrile/water-0.1% trifluoroacetic acid, UV detection at 220 nm); Amino Acid Analysis: Asp(1.97), Gly(2.00), Phe(1.95), Arg(1.03).

### Example 31

### Preparation of (2R,3S)-3-phenylcysteine

### a) (4S)-3-(((4'S,5'R)-5'-phenyl-2'-thioxo-4'-oxazolidinyl) carbonyl)-4-(phenylmethyl)-2-oxazolidinone(1)

To a -78°C suspension of freshly prepared stannous triflate (washed under argon with anhydrous ether three times previously) (7.2 g, 17.6 mmol) and N-ethylpiperidine in dry THF (60 mL) 3-(isothiocyanoacetyl)-2-oxazolidinone (4.4 g, 15.8 mmol) in THF (20 mL) was added. The pale yellow solution was stirred at -78°C for 2 h allowing the solution to warm to - 40°C for 5 min, after cooling again at -78°C, benzaldehyde (2.02 g, 19.1 mmol) was added neat. After the reaction mixture was stirred at -78°C for 2.5 h, it was quenched by the addition of 40 mL of aqueous pH 7 phosphate buffer. The resultant slurry was filtered through Celite®. The filtrate was diluted with 200 mL of 1 N aqueous sodium bisulfate and extracted with CI₂CH₂ (3x). The combined organic phases were dried over anhydrous sodium sulfate and concentrated. Volatiles were removed in vacuo. The residue was dissolved in 200 mL of aqueous sodium bisulfate The residue was purified by flash column chromatography (silica, methylene chloride) to afford the title compound 1 (5.3 g, 87%) . ¹H NMR (CDCl₃) .δ 2.980 (dd, J = 13.6, 8.5 Hz, 1 H, CHHPh), 3.236 (dd, J = 13.6, 3.5 Hz, 1 H, CHHPh), 4.350-4.382 (m, 2 H, H-5), 4.729- 4.823 (m, 1 H, H-4), 4.986 (dd, J = 5.2, 1.9 Hz, 1 H, C(S)NHCH), 6.467 (d, J = 5.2 Hz, 1 H, C(S)OCH), 7.206-7.449 (m, 10 H).

### b) methyl (4S,5R)-5-phenyl-2-thioxooxazolidine-4-carboxylate (2)

To a 0°C solution of aldol product 1 (6.96 g, 18.2 mmol) in anhydrous methanol (42 mL) and Cl₂CH₂ (42 mL) was added via canula a suspension formed by the addition of methylmagnesium bromide (6.7 mL, 20.02 mmol, 1.1 equiv., 3 M in diethyl ether) to anhydrous methanol (25 mL). After the reaction mixture was stirred for 3 min, it was quenched by the addition of 50 mL of 1 N aqueous sodium bisulfate. Volatiles were removed in vacuo. The residue was dissolved in 200 mL of aqueous sodium bisulfate and extracted with of Cl₂CH₂ (3x). The combined organic phases were dried over anhydrous sodium sulfate and concentrated. The residue was purified by flash column chromatography (silica, 5:5:1 methylene chloride/ether/methanol) to afford of the title compound (4.18 g , 96%) 2. ¹H NMR (CDCl₃) .δ 3.875 (s, 3 H), 4.534 (d, J = 6.1 Hz, 1 H, H-4), 5.967 (d, J = 6.1 Hz, 1 H, H-5), 7.414 (s, 5 H); ¹³C NMR (CDCl₃) .δ 53.4, 64.6, 85.6, 125.6, 129.1, 129.5, 136.7, 168.5, 188.8.

### c) methyl (4S,5R)3-((tert-butyloxycarbonyl)- 5-phenyl-2-oxazolidine-4-carboxylate (3)

To a well-stirred solution of the thiooxazolidinone 2 (4.18 g, 17.6 mmol) in Cl₂CH₂ (80 mL) at room temperature di-t-butyl pyrocarbonate (4.25 g, 19.36 mmol) and dimethylaminopyridine (110 mg, 0.05 equiv.) were added dissolved in Cl₂CH₂ (8 mL). After the reaction mixture was stirred for 30 min, it was cooled to 0°C and 30% aqueous H₂0₂ (44 mL) and 88% formic acid (44 mL) were added. The resultant two-phase mixture was stirred for 30 min and then poured into 1 M aqueous potassium carbonate (500 mL) . The aqueous solution was extracted with Cl₂CH₂ (3x). The combined organic phases were washed with 1 M aqueous potassium carbonate, dried over anhydrous sodium sulfate, and concentrated to give a foamy oil (5.6 g, 100%). The oil was purified by a flash column chromatography (30% ethyl acetate/hexane) to give the desired Boc protected oxazolidinone 3 (5.4 g, 95%). ¹H NMR (CDCl₃) .δ 1.504 (s, 9 H), 3.385 (s, 3 H), 4.642 (d, J = 4.3 Hz, 1 H, H-4), 5.389 (d, J = 4.3 Hz, 1 H, H-5), 7.401-7.462 (m, 5H); ¹³C NMR (CDCl₃) .δ 28.2, 53.3, 63.8, 76.0, 84.9, 125.2, 129.3, 129.6, 137.3, 150.8, 169.1.

### d) methyl (2S,3R)2-((tert-butyloxycarbonyl)amino)-3-hydroxy-3-phenylpropionate (4)

To a room temperature solution of methyl ester 3 (5.6 g, 17.6 mmol) in dioxane (300 mL) was added freshly prepared 2 N aqueous lithium hydroxide solution (44 mL, 88 mmol). The resultant suspension was stirred at room temperature overnight. Volatiles were removed in vacuo. The residue was dissolved in 1 N aqueous sodium bisulfate (300 mL) and extracted with Cl₂CH₂ (3x). The combined organic phases were dried over sodium sulfate and concentrated, the residue was then redissolved in ether (300 mL) and, after cooling to 0°C, a solution of ethereal diazomethane was added until a pale yellow color persisted. The solution was stirred at 0°C for 15 min and at room temperature for 30 min. The solvent was eliminated and the residual foamy oil was purified by flash column chromatography (silica, step gradient: 30% ethyl acetate-hexane, 50% ethyl acetate/hexane) to give the desired Boc protected amino alcohol 4 (4.01 g, 81%) followed by the de-Boc oxazolidinone (460 mg, 12%). The amino alcohol 4 was obtained as a white solid, which crystallized. mp 101-102°C (ether-hexane); [α]²⁰_{D} = -15.1° (c = 0.93, CHCl₃); ¹H NMR (CDCl₃) .δ 1.333 (s, 9H), 2.704 (bs, 1 H, OH), 3.761 (s, 3 H), 4.501 (bd, J = 6.6 Hz, 1 H, HO-), 5.237 (dd, J = 3.5, 3.4 Hz, 1 H), 5.273-5.351 (m, 1 H), 7.287-7.362 (m, 5H) ; ¹³C NMR (CDCl₃) .δ 28.1, 52.4, 59.5, 73.8, 80.0, 126.0, 127.9, 128.2, 139.9, 155.5, 171.5; IR (KBr) 3440 (br), 2980, 1745, 1705, 1525, 1165 cm⁻¹; MS m/z 296 [M+H]⁺; Anal. Calcd. for C₁₅H₂₁O₅N: C, 61.02; H, 7.12. Found: C, 59.96; H, 6.94.

### e) methyl (2S,3R)-2-((tert-butyloxycarbonyl)amino)-3-methanesulfonyl-3-phenylpropionate (6)

To a solution of alcohol 4 (0.94 g, 3.18 mmol) and NEt₃ (0.73 g, 7.18 mmol) at 0°C in Cl₂CH₂ (10 mL), methanesulfonyl chloride (418 mg, 3.65 mmol) was added. The reaction mixture was allowed to stir at 0°C for 45 min. It was then quenched with cold dilute HCl, diluted with Cl₂CH₂ and the organic layer was washed with an aqueous solution of NaHCO₃ followed by brine and dried over Na₂SO₄. The solvent was eliminated in vacuo and the crude mesylate 6 was obtained as a foamy white solid which was used in the next step without further purification. ¹H NMR (CDCl₃) .δ 1.417 (s, 9 H), 2.910 (s, 3 H, CH₃-OMs), 3.705 (s, 3 H, CH₃O-), 4.853 (dd, J = 9.6, 6.6 Hz, 1 H, H-C₂), 5.173 (bd, J = 9.6 Hz, 1 H, H-N), 5.893 (d, J = 6.6 Hz, 1 H, H-C₃), 7.389-7.400 (m, 5H); IR (KBR) 3400, 2980, 2950, 1745, 1715, 1520, 1360, 1180 cm⁻¹.

### f) methyl (2R,3S)3-(acetylthio)2-((tert-butyloxycarbonyl)amino)-3-phenylpropionate (7)

To a solution of mesylate 6 (1.18 g, 3.16 mmol) in DMF (10 mL) a preformed solution of the DBU salt of thiolacetic acid (formed by adding thiolacetic acid (1.2 g, 15.8 mmol) to a solution of DBU (1.68 g, 11.07 mmol) in DMF (5 mL)) was added. The reaction mixture was allowed to stir at room temperature for 30 h. It was then quenched with water diluted with Cl₂CH₂ and washed with water (4x). The organic layer was washed with brine and dried over Na₂SO₄. The solvent was removed in vacuo and the crude oil was purified by flash column chromatography (silica, 8% ethyl acetate/toluene to give the desired acetylated thiol 7 (0.97 g, 87%) as a colorless liquid. [α]2²⁰_{D} = +143.6° (c = 2.50, CHCl₃); ¹H NMR (CDCl₃) .δ 1.427 (s, 9 H), 2.330 (s, 3 H, CH₃-COS), 3.685 (s, 3 H, CH₃O-), 4.903 (dd, J = 9.6, 4.7 Hz, 1 H, H-C₂), 5.073 (bd, J = 9.6 Hz, 1 H, H-N), 5.138 (d, J = 4.7 Hz, 1 H, H-C₃), 7.289-7.400 (m, 5H); ¹³C NMR (CDCl₃) .δ 28.1, 30.2, 49.9, 52.2, 57.3, 80.2, 128.2, 128.4, 128.6, 136.6, 155.1, 170.1, 193.3 (-COS-); IR (neat) 3380, 2990, 2960, 1750, 1690, 1520, 1340, 1170 cm⁻l; MS m/z 354 [M+H]⁺; Anal. Calcd. for C₁₇H₂₃O₅NS: C, 57.79; H, 6.51. Found: C, 57.64; H, 6.59.

### g) methyl (2R,3S)-2-((tert-butyloxycarbonyl)amino)-3-mercapto-3-phenylpropionate (9)

The acetylated thiol 7 (290 mg, 0.82 mmol) was dissolved in methanol (4 mL) and NaOH was added (4.1 mL, 0.2 N aqueous). The reaction was stirred at room temperature for 20 min (monitored by TLC). The solution was then carefully neutralized with dilute HCl. The volatiles were removed in vacuo and the residue was extracted with Cl₂CH₂ (3x). The organic layer was washed with brine and dried over Na₂SO₄. The crude product was purified by flash column chromatography (silica, 10% ethyl acetate/hexane) to give the desired thiol 9 (242 mg, 95%) as a white solid. mp 76-77°C (ether-hexane); [α]²⁰_{D} = +92.3 (c = 0.96, CHCl₃); ¹H NMR (CDCl₃) .·δ 1.392 (s, 9 H), 2.207 (d, J = 7.3 Hz, SH), 3.698 (s, 3 H), 4.471 (dd, J = 6.7, 6.3 Hz, 1 H, CH-S), 4.800 (dd, J = 8.7, 6.3 Hz, 1 H, CH-N), 5.059 (bd, J = 8.7 Hz, 1 H, NH), 7.370-7.282 (m, 5H); ¹³C NMR (CDCl₃).δ 28.0, 52.1, 59.5, 80.1, 127.5, 127.6, 128.8, 138.8, 155.9, 171.5; IR (KBr) 3335, 2990, 1740, 1685, 1530, 1165 cm⁻¹; MS m/z 312 [M+H]⁺;. Anal. Calcd. for C₁₅H₂₁O₄NS: C, 57.88; H, 6.75. Found: C, 58.08; H, 6.88.

### h) (2R,3S)-2-((tert-butyloxycarbonyl)amino)-3-((4-methylbenzyl) thio)-3-phenylpropionic acid (10)

The acetylated thiol 7 (520 mg, 1.47 mmol) was dissolved in methanol (8 mL) and NaOH was added (1.62 mL, 1 M aqueous), followed by bromoxilene (300 mg, 1.62 mmol). After 20 min the pH was checked and an additional 1.1 equivalents of base were added. The reaction was allowed to stir at room temperature for 3 h (monitored by TLC). The solution was carefully neutralized with dilute HCl. The volatiles were removed in vacuo and the residue was extracted with Cl₂CH₂ (3x). The organic layer was washed with brine and dried over Na₂SO₄. The crude product was purified by flash column chromatography (silica, 2% acetic acid/20% ethyl acetate/hexane) to give the desired acid 10 (480 mg, 79%) as a white solid. mp 131-132°C (ether-hexane); [α]²⁰_{D} = +211.7° (c = 0.99, CHCl₃); ¹H NMR (CDCl₃) .δ 1.403 (s, 9 H), 2.316 (s, 3 H), 3.515 (d, J = 13 Hz, 1 H), 4.194 (d, J = 13 Hz, 1 H), 4.790 (bs, 1 H), 4.931-5.050 (m, 1 H), 5.050-5.352 (bs, 1 H), 7.082 (s, 4 H), 7.287-7.307 (m, 5 H); IR (KBr) 3380, 3000, 1700, 1515, 1170 cm⁻¹. MS m/z 402 [M+H]⁺;. Anal. Calcd. for C₂₂H₂₇O₄NS: C, 65.81; H, 6.78. Found: C, 65.91; H, 7.00.

### Example 32

### Preparation of (2R,3R)-3-phenylcysteine:

### a) (4R)-3-((2'R,3'S)-2'-bromo-3'-hydroxy-3'-phenylpropanoyl)4-(phenylmethyl)-2-oxazolidinone (12)

To a -78°C suspension of 3-(bromoacetyl)-2-oxazolidinone 11 (4.74 g, 15.9 mmol) in diethyl ether (30 mL) were added trietylamine (2.25 g, 22.2 mmol) and freshly distilled di-n-butylboryl triflate. The cooling bath was removed and the solution was stirred at room temperature for 2 h. The resultant two-phase brown mixture was gradually cooled to -78°C and benzaldehyde was added neat (1.27 g, 11.9 mmol). After the reaction mixture was stirred at -78°C for 30 min and 0°C for 2.5 h, it was diluted with ether, washed with 1 N aqueous sodium bisulfate (2x) and water, and concentrated. The residue was dissolved in ether (30 mL) and cooled to 0°C. The residue was added dropwise to mixture of methanol and 30% aqueous hydrogen peroxide (1:1, 30 mL). The reaction mixture was stirred at 0°C for 1 h, then carefully poured into saturated aqueous sodium bicarbonate, and extracted with ether (2x). The combined organic phases were washed with saturated aqueous sodium bicarbonate (2x), dried over sodium sulfate, and concentrated to give a white solid, which was purified by flash column chromatography (silica, 2% ethyl acetate/methylene chloride) to yield the bromohydrine 12 (3.62 g, 75%).

### b) (4R)-3-((2'S,3'S)-2'-azido-3'-hydroxy-3'-phenylpropanoyl)-4-(phenylmethyl)-2-oxazolidinone (13)

A solution of aldol adduct 12 (3.1 g, 7.67 mmol) and sodium azide (0.99 g, 15.3 mmol) in DMSO (26 mL) was stirred at room temperature for 5 h. The resultant dark solution was diluted with a 2:1 hexane/methylene chloride, washed with water (4x), dried over sodium sulfate, and concentrated to a pale oil, which crystallized. Purification by recrystallization from ether/hexane gave the azide 13 (2.2 g, 80 %) as a white solid, which crystallized. mp 115-116°C (ether-hexane); [α]²⁰_{D} = -6.6^{o} (c = 2.2, CHCl₃); ¹H NMR (CDCl₃) .δ 2.745 (dd, J = 13.6, 9.6 Hz, 1 H, CHH-Ph), 2.949 (d, J = 6.6 Hz, 1 H, -OH), 3.310 (dd, J = 13.6, 3.4 Hz, 1 H, CHHph), 4.199-4.274 (m, 2 H, H-5) 4.714-4.752 (m, 1 H, H-4), 5.075 (dd, J = 8.5, 6.6 Hz, 1 H, CH-OH), 5.383 (d, J= 8.5 Hz, 1 H, CHN₃), 7.197-7.532 (m, 10 H). ¹³C NMR (CDCl₃) .δ 37.3, 55.5, 63.1, 66.5, 74.8, 126.7, 127.3, 128.7, 128.8, 128.9, 129.3, 134.8, 139.5, 153.3, 169.6. IR (KBr) 3420, 3025, 2100, 2760, 1700, 1400, 1225 cm⁻¹. MS m/z 349 [M+H-H₂O]⁺. Anal. Calcd. C₁₉H₁₈O₄N₄: C, 62.29; H, 4.95; N, 15.29. Found: C, 62.03; H, 5.06; N, 15.23.

### c) methyl (2S,3S)-2-azido-3-hydroxy-3-phenylpropionate (14)

To a 0°C solution of the azide 13 (1.52 g, 4.15 mmol) in anhydrous methanol (8 mL) and Cl₂CH₂ (8 mL), a suspension formed by the addition of methylmagnesium bromide (5.2 mL, 4.5 mmol, 0.88 M in diethyl ether) was added via canula to anhydrous methanol (5 mL). After the reaction mixture was stirred for 3 min, it was quenched by the addition of 20 mL of 1 N aqueous sodium bisulfate and extracted with methylene chloride (3x). The combined organic phases were dried over anhydrous sodium sulfate and concentrated. The residue was purified by flash column chromatography (silica, methylene chloride) to afford the title compound 14 (807 mg, 88%) as a white solid, which crystallized. mp 40-41°C (ether-hexane); ¹H NMR (CDCl₃) .δ 3.084 (bs, 1 H, -OH), 3.774 (s, 3 H), 4.108 (d, J = 7 Hz, 1 H, CH-N₃), 5.003 (bd, J = 6.4 Hz, 1 H, HC-OH), 7.372 (s, 5 H); ¹³C NMR (CDCl₃) .δ 52.6, 74.5, 126.6, 128.6, 128.7, 136.1, 169.3; IR (KBr) 3500 (b), 3020, 2960, 2120, 1740, 1460, 1440 cm⁻¹; MS m/z 238 [M+NH₄]; Anal. Calcd. C₁₀H₁₁O₃N₃): C, 54.30; H, 5.01; N, 19.00. Found: C, 54.31; H, 5.08; N, 18.78.

### d) methyl (2S,3S)-2-((tert-butyloxycarbonyl)amino)-3-hydroxy-3-phenylpropionate (15)

Commercially available Palladium on charcoal (80 mg) in ethyl acetate (4 mL) was vigorously stirred under a hydrogen atmosphere for 15 min. To this suspension, a mixture of the azido alcohol 14 (800 mg, 3.6 mmol) and di-t-butyldicarbonate (943 mg, 4.32 mmol) in ethyl acetate (4 mL) was added. The resulting mixture was stirred under hydrogen at room temperature for 2 h (monitored by TLC) and was filtered through a celite®. The filtrate was concentrated in vacuo and the white solid was purified by flash column chromatography (silica, 30% ethyl acetate/hexane) to give the desired Boc protected amino alcohol 15 (1.04 g, 98%) as a white solid, which crystallized. mp 101-102°C (etherhexane); [α]²⁰_{D} = +83.3^{o} (c = 1.05, CHCl₃); ¹H NMR(CDCl₃) .δ 1.433 (s, 9 H), 3.701 (s, 3 H), 3.929 (bd, J = 5.3 Hz, 1 H, HO-), 4.710-4.760 (m, 1 H), 5.168-5.188 (m, 1 H), 5.190-5.310 (m, 1 H), 7.248-7.380 (m, 5 H); ¹³C NMR (CDCl₃) .δ 28.2, 52.2, 59.7, 74.9, 80.5, 126.0, 127.9, 128.2, 139.3, 156.1, 170.1; IR (KBr) 3450, 3390, 3000, 1760, 1710, 1530, 1280, 1260 cm⁻¹; MS m/z 296 [M+H]⁺; Anal. Calcd. for C₁₅H₂₁O₅N: C, 61.02; H, 7.12. Found: C, 60.96; H, 6.96.

### e) methyl (2S,3S)-2-((tert-butyloxycarbonyl)amino)-3-methanesulfonyl-3-phenylpropionate (16)

To a solution of alcohol 15 (1.94 g, 6.81 mmol) and triethylamine (1.25 g, 12.4 mmol) at 0°C in Cl₂CH₂ (15 mL), methanesulfonyl chloride (0.96 g, 8.4 mmol) was added. The reaction mixture was allowed to stir at 0°C for 20 min. It was quenched with cold dilute HCl, diluted with Cl₂CH₂ and the organic layer was washed with an aqueous solution of NaHCO₃ followed by brine and dried over Na₂SO₄. The solvent was removed in vacuo and the crude mesylate 16 was obtained as a foamy white solid, which was used in the next step without further purification. ¹H NMR (CDCl₃).δ 1.394 (s, 9 H), 2.916 (s, 3 H), 3.718 (s, 3 H), 4.878 (dd, J = 8.7, 5.0 Hz, 1 H, H-2), 5.231 (bd, J = 8.7 Hz, 1 H, H-N), 5.917 (d, J = 5.0 Hz, 1 H, H-3), 7.379-7.425 (m, 5 H).

### f) methyl (2R,3R)3-(acetylthio)2-((tert-butyloxycarbonyl) amino)-3-phenylpropionate (17)

To a solution of mesylate 16 (2.72 g, 7.29 mmol) in DMF (7 mL) the potassium salt of thiolacetic acid (3 g, 29.9 mmol) was added at once. The solution became very thick and additional DMF was added (5 mL). The solution was kept under argon at room temperature for 20 h. It was quenched with water diluted with Cl₂CH₂ and washed with water (4x). The organic layer was dried over Na₂SO₄. The solvent was removed in vacuo. The crude product contained a 6:1 mixture of the desired product 17 and oxazolidinone 5 (as indicated by H NMR of the crude). This mixture was purified by flash column chromatography (silica, gradient, 5% to 50% ethyl acetate/hexane) to give the desired acetylated thiol 17 (1.65 g, 69%) as a tan solid followed by oxazolidinone 5 (193 mg, 12%). The acetylated thiol was recrystallized to produce a white solid. 87-88°C (ether-hexane); [α]²⁰_{D} = -97.2° (1.0, CHCl₃); ¹H NMR (CDCl₃) .δ 1.431 (s, 9 H), 2.355 (s, 3 H), 3.616 (s, 3 H), 4.766 (dd, J = 6.6, 6.3 Hz, 1 H; H-2), 5.029 (bd, J = 6.6 Hz, 1 H, H-3), 5.264(bd, J = 6.3 Hz, 1 H, H-N), 7.311 (s, 5 H); ¹³C NMR (CDCl₃) .δ 28.3, 30.2, 50.5, 52.2, 58.5, 80.3, 128.1, 128.3, 128.6, 137.8, 154.9, 170.6, 193.5. IR (KBr) 3380, 2990, 2960, 1745, 1690, 1520, 1240, 1170 cm⁻¹. MS m/z 354[M+H]⁺; Anal. Calcd. C₁₇H₂₃O₅NS: C, 57.77; H, 6.56; N, 3.97. Found: C, 58.05; H, 6.92; N, 3.73.

### g) methyl (2R,3R)-2-((tert-butyloxycarbonyl)amino)-3-((-4-methylbenzyl)thio)-3-phenylpropionic acid (18)

The acetylated thiol 17 (500 mg, 1.4 mmol) was dissolved in methanol (2 mL) and NaOH was added (1.6 mL, 1 M aqueous), followed by bromoxilene (300 mg, 1.62 mmol). After 20 min most of the starting material has disappeared (monitored by TLC). The solution was carefully neutralized with dilute HCl. The volatiles were removed in vacuo and the residue was extracted with Cl₂CH₂ (3x) The organic layer was washed with brine and dried over Na₂SO₄. The crude product was purified by flash column chromatography (silica, 5% to 20% ethyl acetate/hexane) to give the desired ester 18 (480 mg, 79%) as a white solid, which crystallized. mp 97-98°C (etherhexane); [α]²⁰_{D} = -171.8° (c = 1.05, CHCl₃); ¹H NMR (CDCl₃) .δ 1.384 (s, 9 H), 2.326 (s, 3 H), 3.393 (d, J = 13 Hz, 1 H), 3.551 (d, J = 13 Hz, 1 H), 3.572 (s, 3 H, OCH₃), 4.126 (d,J = 5.4 Hz, H-3), 4.630 (dd, J = 8.8, 5.8 Hz, 1 H, H-2), 5.220 (bd, J = 8.8 Hz, 1 H, H-N), 7.028-7.107 (m, 4 H),7.291-7.362 (m, 5 H); ¹³C NMR (CDCl₃) .δ 21.0, 28.3, 35.5, 51.6, 52.1, 57.5, 80.1, 127.8, 128.5, 128.7, 128.9, 129.2, 134.3, 136.9, 138.4, 154.7, 170.8; MS m/z 316 [M+H]⁺; Anal. Calcd for C₂₃H₂₉O₄NS.1/4H₂O: C, 65.76; H, 7.08; N, 3.33. Found: C, 65.59; H, 7.03; N, 3.36.

### h) (2R,3R)-2-((tert-butyloxycarbonyl)amino)-3-((4-methylbenzyl)thio)-3-phenylpropionic acid (19)

The methyl ester 18 (380 mg, 0.91 mmol) and anhydrous lithium chloride (376 mg, 8.89 mmol) were dissolved in dry DMF (10 mL). The reaction mixture was heated at 90°C for 4 days. The reaction mixture was cooled to room temperature, quenched with diluted HCl and extracted several times with ethyl acetate. The organic layer was washed with brine and dried over Na₂SO₄. The crude product was purified by flash column chromatography (silica, step gradient: 20% ethyl acetate/hexane, 25% ethyl acetate/5% acetic acid/hexane) to produce recovered starting material 18 (75 mg, 20%), followed by the desired carboxylic acid as a white solid, which was recrystallized (341 mg, 77%). mp 107-108°C (ether-hexane); [α]²⁰_{D} = -143.7° (c = 1.66, CHCl₃); ¹H NMR (CDCl₃) .δ 1.379 (s, 9 H), 2.300 (s, 3 H), 3.460 (d, J = 13.3 Hz, 1 H), 3.588 (d, J = 13.3 Hz, 1 H), 4.242 (d,J = 5.5 Hz, H-3), 4.646 (dd, J = 8.7, 5.5 Hz, 1 H, H-2), 5.273 (bd, J = 8.7 Hz, 1 H, H-N), 7.055 (s, 4 H), 7.259-7.340 (m, 5 H); ¹³C NMR (CDCl₃) .δ 21.1, 28.2, 35.5, 50.8, 58.1, 80.5, 127.9, 128.6, 128.9, 129.2, 133.9, 136.9, 138.1, 155.3, 174.5; MS m/z 401 ([M+H]⁺; Anal. Calcd. for C₂₂H₂₇O₄NS: C, 65.81; H, 6.78; N, 3.48. Found: C, 65.83; H, 6.92; N, 3.57.

### Example 33

### Parenteral Dosage Unit Composition

A preparation which contains 20 mg of the compound of Example 1 or 2 as a sterile dry powder is prepared as follows: 20 mg of the compound is dissolved in 15 ml of distilled water. The solution is filtered under sterile conditions into a 25 ml multi-dose ampoule and lyophilized. The powder is reconstituted by addition of 20 ml of 5% dextrose in water (D5W) for intravenous or intramuscular injection. The dosage is thereby determined by the injection volume. Subsequent dilution may be made by addition of a metered volume of this dosage unit to another volume of D5W for injection, or a metered dose may be added to another mechanism for dispensing the drug, as in a bottle or bag for IV drip infusion or other injection-infusion system.

### Example 34

### Oral Dosage Unit Composition

A capsule for oral administration is prepared by mixing and milling 50 mg of the compound of Example 3 with 75 mg of lactose and 5 mg of magnesium stearate. The resulting powder is screened and filled into a hard gelatin capsule.

### Example 35

### Oral Dosage Unit Composition

A tablet for oral administration is prepared by mixing and granulating 20 mg of sucrose, 150 mg of calcium sulfate dihydrate and 50 mg of the compound of Example 3 with a 10% gelatin solution. The wet granules are screened, dried, mixed with 10 mg starch, 5 mg talc and 3 mg stearic acid; and compressed into a tablet.

The above description fully discloses how to make and use this invention. This invention, however, is not limited to the precise embodiments described herein, but encompasses all modifications within the scope of the claims which follow.

### SEQUENCE LISTING

SEQ ID NO: 1
   SEQUENCE TYPE: peptide
   SEQUENCE LENGTH: 4 amino acids
   TOPOLOGY: circular
   FEATURES:
   Xaa at 1 is N-alpha-2-mercaptobenzoyl Arg
   Xaa at 4 is cysteine amide
   S of Xaa at 1 and S of Xaa at 4 form disulfide
   PROPERTIES:
   fibrinogen receptor antagonist 
Xaa Gly Asp Xaa
1
SEQ ID NO: 2
   SEQUENCE TYPE: peptide
   SEQUENCE LENGTH: 4 amino acids
   TOPOLOGY: circular
   FEATURES:
   Xaa at 1 is N-alpha-acetyl Cys
   Xaa at 4 is 2-mercaptophenylamide of Asp
   S of Xaa at 1 and S of Xaa at 1 form disulfide
   PROPERTIES: fibrinogen receptor antagonist 
Xaa Arg Gly Xaa
1
SEQ ID NO: 3
   SEQUENCE TYPE: peptide
   SEQUENCE LENGTH: 4 amino acids
   TOPOLOGY: circular
   FEATURES:
   Xaa at 1 is N-alpha-methyl,N-alpha-2-mercaptobenzoyl Arg
   Xaa at 4 is beta-phenyl cysteine amide
   S of Xaa at 1 and S of Xaa at 4 form disulfide
   PROPERTIES: fibrinogen receptor antagonist 
Xaa Gly Asp Xaa
1
SEQ ID NO: 4
   SEQUENCE TYPE: peptide
   SEQUENCE LENGTH: 4 amino acids
   TOPOLOGY: circular
   FEATURES:
   Xaa at 1 is N-alpha-2-mercaptobenzoyl MeGly
   Xaa at 4 is 2-mercaptophenyl amide of Asp
   S of Xaa at 1 and S of Xaa at 4 form disulfide
   PROPERTIES: fibrinogen receptor antagonist 
Xaa Arg Gly Xaa
1
SEQ ID NO: 5
   SEQUENCE TYPE: peptide
   SEQUENCE LENGTH: 4 amino acids
   TOPOLOGY: circular
   FEATURES:
   Xaa at 1 is N-alpha-2-mercaptobenzoyl MeGly
   Xaa at 2 is N-alpha-methyl Arg
   Xaa at 4 is 2-mercaptophenyl amide of Asp
   S of Xaa at 1 and S of Xaa at 4 form disulfide
   PROPERTIES: fibrinogen receptor antagonist 
Xaa Xaa Gly Xaa
1
SEQ ID NO: 6
   SEQUENCE TYPE: peptide
   SEQUENCE LENGTH: 5 amino acids
   TOPOLOGY: circular
   FEATURES:
   Xaa at 1 is N-alpha-acetyl Cys
   Xaa at 2 is N-alpha-methyl Arg
   Xaa at 5 is (3S)beta-phenyl-cysteine amide
   S of Xaa at 1 and S of Xaa at 5 form disulfide
   PROPERTIES: fibrinogen receptor antagonist 
Xaa Xaa Gly Asp Xaa
1               5
SEQ ID NO: 7
   SEQUENCE TYPE: peptide
   SEQUENCE LENGTH: 5 amino acids
   TOPOLOGY: circular
   FEATURES:
   Xaa at 1 is N-alpha-acetyl Cys
   Xaa at 2 is N-alpha-methyl Arg
   Xaa at 5 is (3R)beta-phenyl-cysteine amide
   S of Xaa at 1 and S of Xaa at 5 form disulfide
   PROPERTIES: fibrinogen receptor antagonist 
Xaa Xaa Gly Asp Xaa
1               5
SEQ ID NO: 10
   SEQUENCE TYPE: peptide
   SEQUENCE LENGTH: 10 amino acids
   TOPOLOGY: circular
   FEATURES:
   Xaa at 1 is N-alpha-acetyl Cys
   Xaa at 6 is N-alpha-methyl Arg
   Xaa at 10 is cysteine amide
   S of Xaa at 1 and S of Xaa at 10 form disulfide
   PROPERTIES: fibrinogen receptor antagonist 
Xaa Arg Gly Asp Ser Xaa Gly Asp Ser Xaa
1               5                   10
SEQ ID NO: 11
   SEQUENCE TYPE: peptide
   SEQUENCE LENGTH: 10 amino acids
   TOPOLOGY: circular
   FEATURES:
   Xaa at 1 is N-alpha-acetyl Cys
   Xaa at 2 is N-alpha-methyl Arg
   Xaa at 10 is cysteine amide
   S of Xaa at 1 and S of Xaa at 10 form disulfide
   PROPERTIES: fibrinogen receptor antagonist 
Xaa Xaa Gly Asp Ser Arg Gly Asp Ser Xaa
1               5                   10
SEQ ID NO: 14
   SEQUENCE TYPE: peptide
   SEQUENCE LENGTH: 6 amino acids
   TOPOLOGY: circular
   FEATURES:
   Xaa at 2 is N-alpha-methyl Arg
   Xaa at 6 is glutamic acid amide
   alpha-amino group of Gly at 1 forms peptide bond with gamma-carboxyl group of Xaa at 6
   PROPERTIES: fibrinogen receptor antagonist 
Gly Xaa Gly Asp Ser Xaa
1               5
SEQ ID NO: 16
   SEQUENCE TYPE: peptide
   SEQUENCE LENGTH: 8 amino acids
   TOPOLOGY: circular
   FEATURES:
   Xaa at 1 is alpha-methyl Arg
   alpha-amino group of Xaa at 1 forms peptide bond with carboxyl group of Phe at 8
   PROPERTIES: fibrinogen receptor antagonist 
Xaa Gly Asp Phe Arg Gly Asp Phe
1               5
SEQ ID NO: 18
   SEQUENCE TYPE: peptide
   SEQUENCE LENGTH: 5 amino acids
   TOPOLOGY: circular
   FEATURES:
   Xaa at 1 is N-alpha-acetyl Cys
   Xaa at 2 is N-alpha-methyl Arg
   Xaa at 5 is beta-phenyl-cysteine
   S of Xaa at 1 and S of Xaa at 5 form disulphide
   PROPERTIES: fibrinogen receptor antagonist

Xaa Xaa Gly Asp Xaa
1               5

## Claims

1. A compound of the formula: 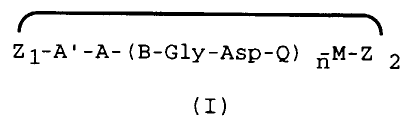
Z1-A'-A-(B-Gly-Asp-Q)n-M-Z2
(I)
 wherein:
A' is absent, Asn, Gln, Ala or Abu;
A is absent or a D- or L- amino acid chosen from Arg, HArg, NArg, (Me₂)Arg, (Et₂)Arg, Abu, Ala, Gly, His, Lys, or an α-R' substituted derivative thereof, Dtc, Tpr or Pro;
B is a D- or L- amino acid chosen from Arg, HArg, (Me₂)Arg, (Et₂)Arg, Lys or an α-R' substituted derivative thereof;
Q is absent or a D or L amino acid chosen from Tyr, (Alk)Tyr, Phe, (4'W)Phe, HPhe, Phg, Pro, Trp, His, Ser, (Alk)Ser, Thr, (Alk)Thr, (Alk)Cys, (Alk)Pen, Ala, Val, Nva, Met, Leu, Ile, Nle or Nal, or an α-R' substituted derivative thereof;
M is absent or Gly or D or L Glu, Phe, Pro, Lys, Ser or, provided n is 1, B-Gly-Glu-Q;
W is halogen or Alk;
R' is Alk or PhCH₂;
Z₁ is 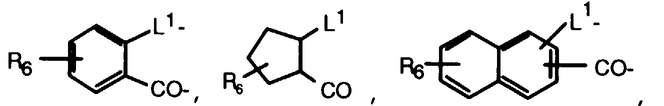 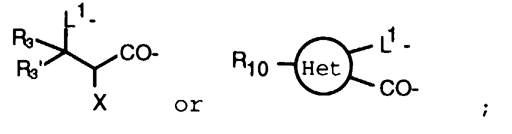
Z₂ is 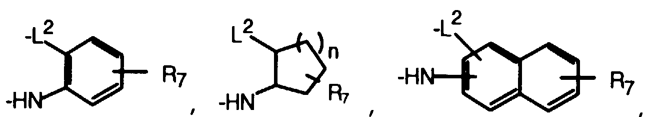 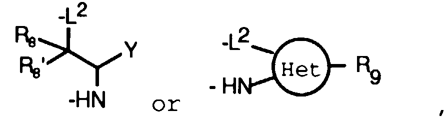 wherein Z₁ and Z₂ are linked via a covalent bond between L¹ and L²;
L¹ and L² are -S-;
X is R₄R₅N;
Y is H, CONR₁R₂ or CO₂R₂;
R₁ and R₂ are H, Alk or (CH₂)ₚAr;
R₃ and R_{3'} are H, Alk, (CH₂)ₚAr or taken together are -(CH₂)₄- or -(CH₂)₅-;
R₄ is H or Alk;
R₅ is R₁₁, R₁₁CO, R₁₁OCO, R₁₁OCH(R_{11'})CO, R₁₁NHCH(R_{11'})CO, R₁₁SCH(R_{11'})CO, R₁₁SO₂ or R₁₁SO;
R₆ is Alk, OAlk, halogen or X;
R₇ is H, Alk, OAlk, halogen or Y;
R₈ and R_{8'} are H, Alk, (CH₂)ₚPh, (CH₂)ₚNph or taken together are -(CH₂)₄- or -(CH₂)₅-;
R₉ is H, Alk or Y;
R₁₀ is H or Alk;
R₁₁ and R_{11'} are H, C₁₋₅alkyl, C₃₋₇cycloalkyl, Ar, Ar-C₁₋₅alkyl, Ar-C₃₋₇cycloalkyl;
Ar is phenyl or phenyl substituted by one or two C₁₋₅alkyl, trifluoromethyl, hydroxy, C₁₋₅alkoxy or halogen groups;
Alk is C₁₋₄alkyl;
Het is a heterocycle selected from pyridine, pyrrole, pyrrolidine, imidazole, triazole, thiophene, furan and thiazole, wherein L¹ is ortho to -CO- and L² is ortho to -NH-;
n is 1 or 2; and
p is 0, 1, 2 or 3
and pharmaceutically active salts thereof;
provided that when Z₁ is -L¹-C(R₃)(R_{3'})-CH(X)-CO-, R₃ and R_{3'} are H or Alk or together are -(CH₂)₄- or -(CH₂)₅- and Z₂ is -L²-C(R₈)(R_{8'})-CH(Y)-NH-, R₈ and R_{8'} are not both H or Alk nor together are -(CH₂)₄- or -(CH₂)₅-; and provided that M is absent only when Q is absent and only when Z₂ is

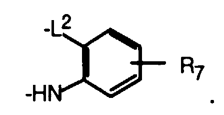

2. A compound according to claim 1 in which Z₁ is 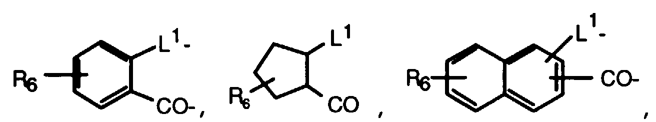 or

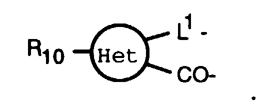

3. A compound according to claim 1 in which Z₂ is 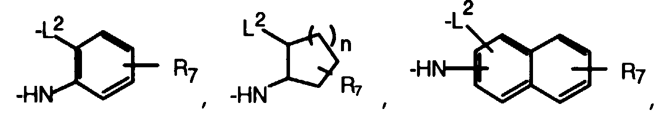 or

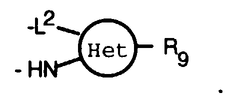

4. A compound according to claim 1 in which Z₁ is 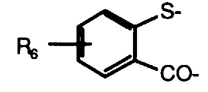 or Cys
and Z₂ is 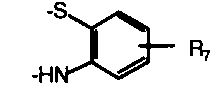 or Pcs.

5. A compound according to any of claims 1 to 4 in which Z₁ is

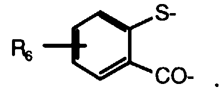

6. A compound according to any of claims 1 to 5 in which Z₂ is

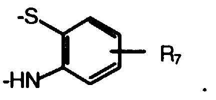

7. A compound according to any of claims 1 to 6 in which B is Arg, HArg or an α-R' substituted derivative of Arg or HArg.

8. A compound according to any of claims 1 to 7 in which A' and A are absent.

9. A compound according to any of claims 1 to 8 in which B is MeArg.

10. A compound which is:
cyclo(S,S)-Mba-Arg-Gly-Asp-Cys-NH₂ (SEQ ID NO:1);
N^{α}-Ac-cyclo(S,S)-Cys-Arg-Gly-Asp-Man (SEQ ID NO:2);
cyclo (S,S)-Mba-MeArg-Gly-Asp-Man;
cyclo(S,S)-Mba-MeArg-Gly-Asp-Pcs-NH₂ (SEQ ID NO:3);
cyclo-(S,S)-Mba-Sar-Arg-Gly-Asp-Man (SEQ ID NO:4);
cyclo-(S,S)-Mba-Sar-MeArg-Gly-Asp-Man (SEQ ID NO:5);
cyclo-(S,S)-Mba-Arg-Gly-Asp-Man;
cyclo-(S,S)-Mba-D-MeArg-Gly-Asp-Man;
N^{α}Ac-cyclo-(S,S)-Cys-MeArg-Gly-Asp-(2R,3S)Pcs-NH₂ (SEQ ID NO:6);
N^{α}Ac-cyclo-(S,S)-Cys-MeArg-Gly-Asp-(2R,3R)Pcs-NH₂ (SEQ ID NO:7);
N^{α}Ac-cyclo-(S,S) Cys-Arg-Gly-Asp-Ser-MeArg -Gly-Asp-Ser-Cys-NH₂ (SEQ ID NO:10);
N^{α}Ac-cyclo-(S,S)-Cys-MeArg-Gly-Asp-Ser-Arg -Gly-Asp-Ser-Cys-NH₂ (SEQ ID NO:11);

11. A pharmaceutical composition which comprises a compound according to any of claims 1 to 10, 19 or 20 and a pharmaceutically acceptable carrier.

12. A compound according to any one of claims 1 to 10, 19 or 20 for use in a medicament.

13. The use of a compound according to any one of claims 1 to 10, 19 or 20 in the manufacture of a medicament for the treatment of acute myocardial infarction.

14. The use of a compound according to any one of claims 1 to 10, 19 or 20 in the manufacture of a medicament for the treatment of stroke or transient ischemia attacks.

15. The use of a compound according to any one of claims 1 to 10, 19 or 20 in the manufacture of a medicament for treating unstable angina.

16. The use of a compound according to any one of claims 1 to 10, 19 or 20 and a fibrinolytic agent in the manufacture of a medicament for effecting thrombolysis and inhibiting reocclusion of an artery or vein in a mammal.

17. A compound of the formula: 
S-T1                                    S-T2
|                                        |
Z1-A'-A-(B-Gly-Asp-Q) n-M-Z2

(II)
 wherein:
A' is absent, Asn, Gln, Ala or Abu;
A is absent or a D- or L- amino acid chosen from Arg, HArg, (Me₂)Arg, (Et₂)Arg, Abu, Ala, Gly, His, Lys, or an α-R' substituted derivative thereof, Dtc, Tpr or Pro;
B is a D- or L- amino acid chosen from Arg, HArg, NArg, (Me₂)Arg, (Et₂)Arg, Lys or an α-R' substituted derivative thereof;
Q is absent or a D or L amino acid chosen from Tyr, (Alk)Tyr, Phe, (4'W)Phe, HPhe, Phg, Pro, Trp, His, Ser, (Alk)Ser, Thr, (Alk)Thr, (Alk)Cys, (Alk)Pen, Ala, Val, Nva, Met, Leu, Ile, Nle or Nal, or an α-R' substituted derivative thereof;
M is absent or Gly or D or L Glu, Phe, Pro, Lys, Ser or, provided n is 1, B-Gly-Glu-Q;
W is halogen or Alk;
R' is Alk or PhCH₂;
Z₁ is 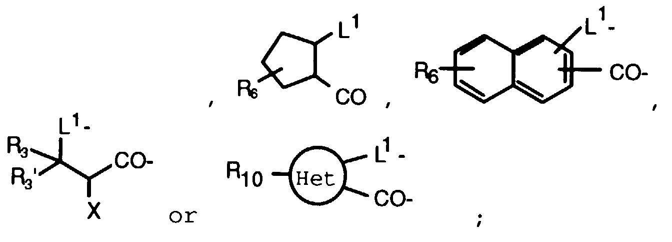
Z₂ is 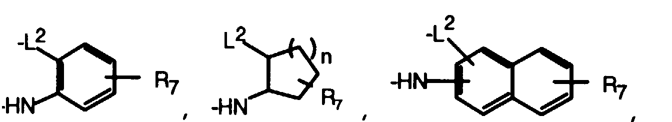 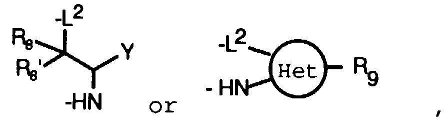 wherein Z₁ and Z₂ are linked via a covalent bond between L¹ and L²;
L¹ and L² are -S-;
T¹ and T² are each hydrogen or a displaceable group;
X is R₄R₅N;
Y is H, CONR₁R₂ or CO₂R₂;
R₁ and R₂ are H, Alk or (CH₂)ₚAr;
R₃ and R_{3'} are H, Alk, (CH₂)ₚAr or taken together are -(CH₂)₄- or -(CH₂)₅-;
R₄ is H or Alk;
R₅ is R₁₁, R₁₁CO, R₁₁OCO, R₁₁OCH(R_{11'})CO, R₁₁NHCH(R_{11'})CO, R₁₁SCH(R_{11'})CO, R₁₁SO₂ or R₁₁SO;
R₆ is Alk, OAlk, halogen or X;
R₇ is H, Alk, OAlk, halogen or Y;
R₈ and R_{8'} are H, Alk, (CH₂)ₚPh, (CH₂)ₚNph or taken together are -(CH₂)₄- or -(CH₂)₅-;
R₉ is H, Alk or Y;
R₁₀ is H or Alk;
R₁₁ and R_{11'} are H, C₁₋₅alkyl, C₃₋₇cycloalkyl, Ar, Ar-C₁₋₅alkyl, Ar-C₃₋₇cycloalkyl;
Ar is phenyl or phenyl substituted by one or two C₁₋₅alkyl, trifluoromethyl, hydroxy, C₁₋₅alkoxy or halogen groups;
Alk is C₁₋₄alkyl;
Het is a heterocycle selected from pyridine, pyrrole, pyrrolidine, imidazole, triazole, thiophene, furan and thiazole, wherein L¹ is ortho to -CO- and L² is ortho to -NH-;
n is 1 or 2; and
p is 0, 1, 2 or 3
provided that when Z₁ is -L¹-C(R₃)(R_{3'})-CH(X)-CO-, R₃ and R_{3'} are H or Alk or together are -(CH₂)₄- or -(CH₂)₅- and Z₂ is -L²-C(R₈)(R_{8'})-CH(Y)-NH-, R₈ and R_{8'} are not both H or Alk nor together are -(CH₂)₄- or -(CH₂)₅-; and
provided that M is absent only when Q is absent and only when Z₂ is

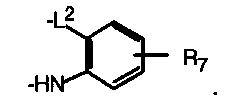

18. A process for preparing a compound of the formula (I) as previously defined in claim 1, which comprises,
a) oxidatively cyclizing a compound of the formula: 
S-T^1                                                    S-T^2
|                                                        |
Z_1-A'-A-(B-Gly-Asp-Q) n-M-Z_2

(II)
 wherein:
A', A, B, Q, M, Z₁, Z₂ and n are as defined in claim 1; and
T¹ and T² are H, or
b) nucleophilically cyclizing a compound of formula (II) by treating with base, wherein A', A, B, Q, M, Z₁, Z₂ and n are as previously defined, and one of T¹ and T² is a displaceable group and the other is H.

19. A compound of the formula: 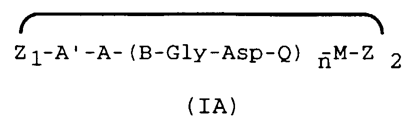
Z1-A'-A-(B-Gly-Asp-Q)n-M-Z2

(IA)
 wherein:
A' is absent, Asn, Gln, Ala or Abu;
A is absent or a D- or L- amino acid chosen from Arg, HArg, NArg, (Me₂)Arg, (Et₂)Arg, Abu, Ala, Gly, His, Lys, or an α-R' substituted derivative thereof, Dtc, Tpr or Pro;
B is a MeArg;
Q is absent or a D or L amino acid chosen from Tyr, (Alk)Tyr, Phe, (4'W)Phe, HPhe, Phg, Pro, Trp, His, Ser, (Alk)Ser, Thr, (Alk)Thr, (Alk)Cys, (Alk)Pen, Ala, Val, Nva, Met, Leu, Ile, Nle or Nal, or an α-R' substituted derivative thereof;
M is Gly or D or L Glu, Phe, Pro, Lys, Ser or,
provided n is 1, B-Gly-Glu-Q;
W is halogen or Alk;
R' is Alk or PhCH₂;
n is 1 or 2;
Alk is C₁₋₄alkyl;
Z₁ and Z₂ are, taken together, a covalent bond between the amino terminal residue and the carboxy terminal residue;
and pharmaceutically active salts thereof.

20. A compound according to claim 19 which is:
cyclo (1^{α}, 6^{γ}) -Gly-MeArg-Gly-Asp-Ser-Glu-NH₂ (SEQ ID NO:14);
cyclo-(1,8)-MeArg-Gly-Asp-Phe-Arg-Gly-Asp-Phe (SEQ ID NO:16).

21. A process for preparing a compound of the formula: 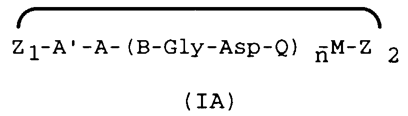
Z1-A'-A-(B-Gly-Asp-Q) n-M-Z2
(IA)
 wherein:
A', A, B, Q, M, and n are as defined in claim 19;
Z₁ and Z₂ are, taken together, a covalent bond between the amino terminal residue and the carboxy terminal residue;
which comprises,
i) cyclizing with a coupling reagent a compound of the formula: H-[A'-A-(B-Gly-Asp-Q-)n-M]-OH wherein H- and -OH represent the amino and carboxyl residues of any two adjacent residues in the cyclic peptide, and A', A, B, Q, M and n are as defined in claim 19 with any reactive groups optionally protected, and
ii) removing any protecting groups.

## Patentansprüche

1. Verbindung der Formel: 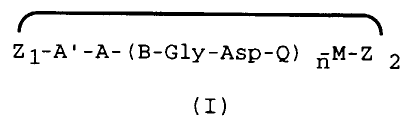
Z1-A'-A-(B-Gly-Asp-Q)n-M-Z2
(I)
 in der:
A' abwesend ist oder Asn, Gln, Ala oder Abu bedeutet;
A abwesend ist oder eine D- oder L-Aminosäure bedeutet, ausgewählt aus Arg, HArg, NArg, (Me₂)Arg, (Et₂)Arg, Abu, Ala, Gly, His, Lys oder einem α-R'-substituierten Derivat davon, Dtc, Tpr oder Pro;
B eine D- oder L-Aminosäure ist, ausgewählt aus Arg, HArg, (Me₂)Arg, (Et₂)Arg, Lys oder einem α-R'-substituierten Derivat davon,
Q abwesend ist oder eine D- oder L-Aminosäure bedeutet, ausgewählt aus Tyr, (Alk)Tyr, Phe, (4'W)Phe, HPhe, Phg, Pro, Trp, His, Ser, (Alk)Ser, Thr, (Alk)Thr, (Alk)Cys, (Alk)Pen, Ala, Val, Nva, Met, Leu, Ile, Nle oder Nal oder einem α-R'-substituierten Derivat davon;
M abwesend ist oder Gly oder D- oder L- Glu, Phe, Pro, Lys, Ser oder, wenn n 1 ist, β-GLy-Glu-Q bedeutet;
W ein Halogenatom oder einen Alk-Rest bedeutet;
R' einen Alk-Rest oder PhCH₂ bedeutet;
Z₁ die folgenden Reste bedeutet 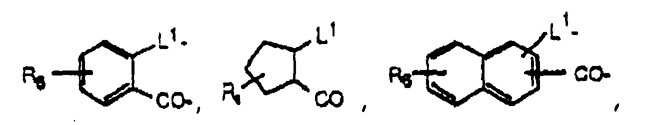 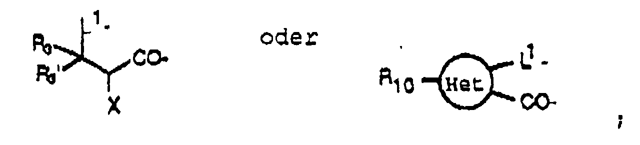
Z₂ die folgenden Reste bedeutet 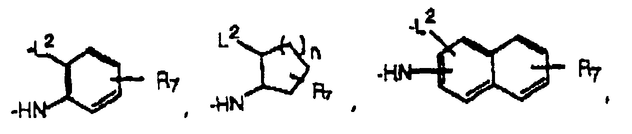 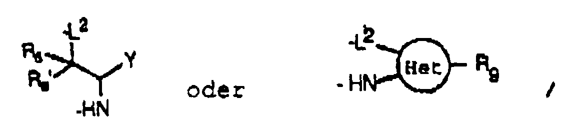 wobei Z₁ und Z₂ über eine kovalente Bindung zwischen L¹ und L² verknüpft sind;
L¹ und L² -S- bedeuten;
X den Rest R₄R₅N bedeutet;
Y ein Wasserstoffatom, einen CONR₁R₂- oder einen CO₂R₂-Rest bedeutet;
R₁ und R₂ Wasserstoffatome, Alk- oder (CH₂)ₚAr-Reste bedeuten;
R₃ und R_{3'} Wasserstoffatome, Alk-, (CH₂)ₚAr-Reste oder zusammengenommen die Gruppen -(CH₂)₄- oder -(CH₂)₅- bedeuten;
R₄ ein Wasserstoffatom oder einen Alk-Rest darstellt;
R₅ die Reste R₁₁, R₁₁CO, R₁₁OCO, R₁₁OCH(R_{11'}) CO, R₁₁NHCH(R_{11'})CO, R₁₁SCH(R_{11'})CO, R₁₁SO₂ oder R₁₁SO bedeutet;
R₆ einen Alk-, OAlk-Rest, ein Halogenatom oder X bedeutet;
R₇ ein Wasserstoffatom, einen Alk-, OAlk-Rest, ein Halogenatom, oder Y bedeutet;
R₈ und R_{8'} Wasserstoffatome, Alk-, (CH₂)ₚPh-, (CH₂)ₚNph-Reste oder zusammengenommen die Gruppen -(CH₂)₄- oder -(CH₂)₅- bedeuten;
R₉ ein Wasserstoffatom, einen Alk-Rest oder Y bedeutet;
R₁₀ ein Wasserstoffatom oder einen Alk-Rest bedeutet;
R₁₁ und R_{11'} Wasserstoffatome, C₁₋₅-Alkyl-, C₃₋₇-Cycloalkyl-, Ar-, Ar-C₁₋₅-alkyl-, Ar-C₃₋₇-cycloalkylreste darstellen;
Ar eine Phenylgruppe oder eine mit einem oder zwei C₁₋₅-Alkyl-, Trifluormethyl-, Hydroxy-, C₁₋₅-Alkoxyresten oder Halogenatomen substituiert ist,
Alk einen C₁₋₄-Alkylrest bedeutet;
Het ein heterocyclischer Rest ist, ausgewählt aus Pyridin, Pyrrol, Pyrrolidin, Imidazol, Triazol, Thiophen, Furan und Thiazol, wobei L¹ sich in ortho-Stellung zu -CO- und L² sich in ortho-Stellung zu -NH- befindet;
n die Bedeutung 1 oder 2 hat; und
p die Bedeutung 0, 1, 2 oder 3 hat und pharmazeutisch aktive Salze davon;
mit der Maßgabe, daß, wenn Z₁ -L¹-C(R₃) (R_{3'})-CH(X)-CO- ist,
R₃ und R_{3'} Wasserstoffatome oder Alk-Reste bedeuten oder zusammen die Gruppen -(CH₂)₄- oder -(CH₂)₅- darstellen, und wenn Z₂ -L²-C(R₈)(R_{8'})-CH(Y)-NH- ist, R₈ und R_{8'} weder beide Wasserstoffatome oder Alkreste sind noch zusammen die Gruppen -(CH₂)₄- oder -(CH₂)₅- bilden; und
mit der Maßgabe, daß M nur dann abwesend ist, wenn Q abwesend ist und wenn Z die folgende Bedeutung hat

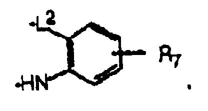

2. Verbindung nach Anspruch 1, in der Z₁ die folgende Bedeutung hat: 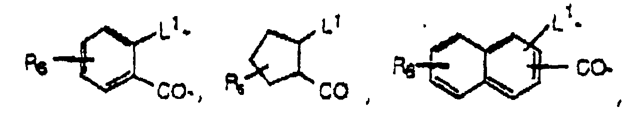 oder

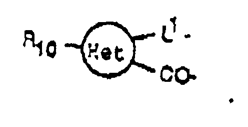

3. Verbindung nach Anspruch 1, in der Z₂ die folgende Bedeutung hat: 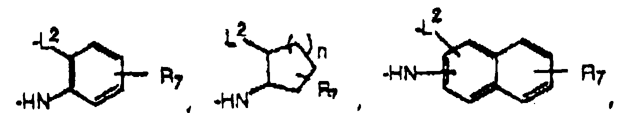 oder

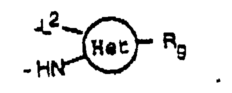

4. Verbindung nach Anspruch 1, in der Z₁ die folgende Bedeutung hat 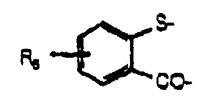 oder Cys und Z₂ die folgende Bedeutung hat 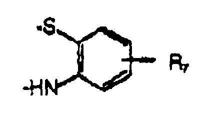 oder Pcs.

5. Verbindung nach einem der Ansprüche 1 bis 4, in der Z₁ die folgende Bedeutung hat

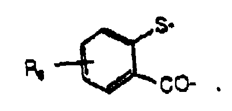

6. Verbindung nach einem der Ansprüche 1 bis 5, in der Z₂ die folgende Bedeutung hat

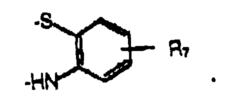

7. Verbindung nach einem der Ansprüche 1 bis 6, in der B Arg, HArg oder ein α-R'-substituiertes Derivat von Arg oder HArg ist.

8. Verbindung nach einem der Ansprüche 1 bis 7, in der A' und A abwesend sind.

9. Verbindung nach einem der Ansprücbe 1 bis 8, in der B MeArg ist.

10. Verbindung, nämlich:
Cyclo(S,S)-Mba-Arg-Gly-Asp-Cys-NH₂ (SEQ ID No.1);
N^{α}-Ac-Cyclo(S,S)-Cys-Arg-Gly-Asp-Man (SEQ ID No. 2);
Cyclo(S,S)-Mba-MeArg-Gly-Asp-Man;
Cyclo(S,S)-Mba-MeArg-Gly-Asp-Pcs-NH₂ (SEQ ID No. 3);
Cyclo-(S,S)-Mba-Sar-Arg-Gly-Asp-Man (SEQ ID No. 4);
Cyclo-(S,S)-Mba-Sar-MeArg-Gly-Asp-Man (SEQ ID No.5);
Cyclo-(S,S)-Mba-Arg-Gly-Asp-Man;
Cyclo-(S,S)-Mba-D-MeArg-Gly-Asp-Man;
N^{α}Ac-Cyclo-(S,S)-Cys-MeArg-Gly-Asp-(2R, 3S)Pcs-NH₂ (SEQ ID No. 6),
N^{α}Ac-Cyclo-(S,S)-Cys-MeArg-Gly-Asp- (2R, 3R)Pcs-NH₂ (SEQ ID No. 7);
N^{α}Ac-Cyclo-(S,S) Cys-Arg-Gly-Asp-Ser-MeArg-Gly-Asp-Ser-Cys-NH₂ (SEQ ID No. 10);
N^{α}Ac-Cyclo-(S,S)-Cys-MeArg-Gly-Asp-Ser-Arg-Gly-Asp-Ser-Cys-NH₂ (SEQ ID No. 11).

11. Arzneimitel, umfassend eine Verbindung nach einem der Ansprüche 1 bis 10, 19 oder 20 und einen pharmazeutisch verträglichen Träger.

12. Verbindung nach einem der Ansprüche 1 bis 10, 19 oder 20 zur Verwendung in einem Medikament.

13. Verwendung einer Verbindung nach einem der Ansprüche 1 bis 10, 19 oder 20 für die Herstellung eines Medikaments zur Behandlung eines akuten Myocardinfarkts.

14. Verwendung einer Verbindung nach einen der Ansprüche 1 bis 10, 19 oder 20 für die Herstellung eines Medikaments zur Behandlung von Schlaganfall oder vorübergehenden ischemischen Attacken.

15. Verwendung einer Verbindung nach einem der Ansprüche 1 bis 10, 19 oder 20 für die Herstellung eines Medikaments zur Behandlung von instabiler Angina.

16. Verwendung einer Verbindung nach einem der Ansprüche 1 bis 10, 19 oder 20 und eines fibrinolytischen Mittels für die Herstellung eines Medikaments zur Erzeugung einer Thrombolyse und Hemmung einer arteriellen oder venösen Restenose in einem Säuger.

17. Verbindung der Formel: 
S-T1                                    S-T2
|                                       |
Z1-A'-A-(B-Gly-Asp-Q) n-M-Z2

(II)
 in der:
A' abwesend ist oder Asn, Gln, Ala oder Abu bedeutet;
A abwesend ist oder eine D- oder L-Aminosäure bedeutet, ausgewählt aus Arg, HArg, (Me₂)Arg, (Et₂)Arg, Abu, Ala, Gly, His, Lys oder einem α-R'-substituierten Derivat davon, Dtc, Tpr oder Pro;
B eine D- oder L-Aminosäure ist, ausgewahlt aus Arg, HArg, NArg, (Me₂)Arg, (Et₂)Arg, Lys oder einem α-R'-substituierten Derivat davon,
Q abwesend ist oder eine D- oder L-Aminosäure bedeutet, ausgewählt aus Tyr, (Alk)Tyr, Phe, (4'W)Phe, HPhe, Phg, Pro, Trp, His, Ser, (Alk)Ser, Thr, (Alk)Thr, (Alk)Cys, (Alk)Pen, Ala, Val, Nva, Met, Leu, Ile, Nle oder Nal oder einem α-R'-substituierten Derivat davon;
M abwesend ist oder Gly oder D- oder L-Glu, Phe, Pro, Lys, Ser oder, wenn n 1 ist, B-Gly-Glu-Q bedeutet ;
W ein Halogenatom oder einen Alk-Rest bedeutet;
R' einen Alk-Rest oder PhCH₂ bedeutet;
Z₁ die folgenden Reste bedeutet 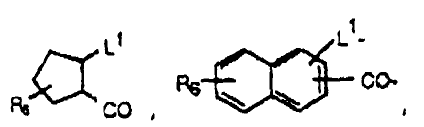 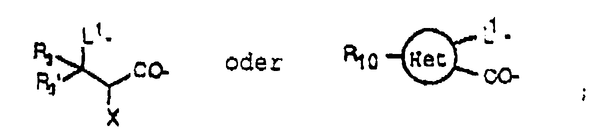
Z₂ die folgenden Reste bedeutet 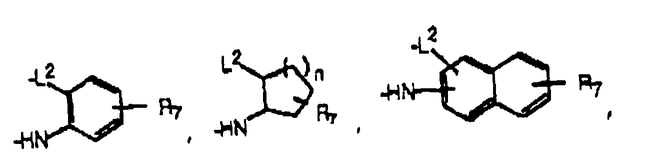 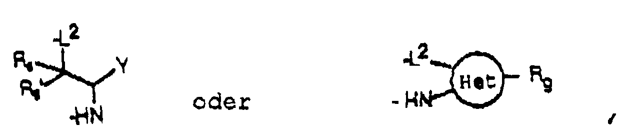
wobei Z₁ und Z₂ uber eine kovalente Bindung zwischen L¹ und L² verknüpft sind;
L¹ und L² -S- bedeuten;
T¹ und T² jeweils ein Wasserstoffatom oder einen ersetzbaren Rest bedeutet;
X den Rest R₄R₅N bedeutet:
Y ein Wasserstoffatom, einen CONR₁R₂- oder einen CO₂R₂-Rest bedeutet;
R₁ und R₂ Wasserstoffatome, Alk- oder (CH₂)ₚAr-Reste bedeuten;
R₃ und R_{3'} Wasserstoffatome, Alk-, (CH₂)ₚAr-Reste oder zusammengenommen die Gruppen -(CH₂)₄- oder -(CH₂)₅- bedeuten;
R₄ ein Wasserstoffatom oder einen Alk-Rest darstellt;
R₅ die Reste R₁₁, R₁₁CO, R₁₁OCO, R₁₁OCH(R_{11'})CO, R₁₁NHCH(R_{11'})CO, R₁₁SCH(R_{11'})CO, R₁₁SO₂ oder R₁₁SO bedeutet;
R₆ einen Alk-, OAlk-Rest, ein Halogenatom oder X bedeutet;
R₇ ein Wasserstoffatom, einen Alk-, OAlk-Rest, ein Halogenatom oder Y bedeutet;
R₈ und R_{8'} Wasserstoffatome, Alk-, (CH₂)ₚPh-, (CH₂)ₚNph-Reste oder zusammengenommen die Gruppen -(CH₂)₄- oder -(CH₂)₅- bedeuten;
R₉ ein Wasserstoffatom, einen Alk-Rest oder Y bedeutet;
R₁₀ ein Wasserstoffatom oder einen Alk-Rest bedeutet;
R₁₁ und R_{11'} Wasserstoffatome, C₁₋₅-Alkyl-, C₃₋₇-Cycloalkyl-, Ar-, Ar-C₁₋₅-alkyl-, Ar-C₃₋₇-cycloalkylreste darstellen;
Ar eine Phenylgruppe oder eine mit einem oder zwei C₁₋₅-Alkyl-, Trifluormethyl-, Hydroxy-, C₁₋₅-Alkoxyresten oder Halogenatomen substituiert ist,
Alk einen C₁₋₄-Alkylrest bedeutet;
Het ein heterccyclischer Rest ist, ausgewählt aus Pyridin, Pyrrol, Pyrrolidin, Imidazol, Triazol, Thiophen, Furan und Thiazol, wobei L¹ sich in ortho-Stellung zu -CO- und L² sich in ortho-Stellung zu -NH- befindet;
n die Bedeutung 1 oder 2 hat; und
p die Bedeutung 0, 1, 2 oder 3 hat;
mit der Maßgabe, daß, wenn Z₁ -L¹-C(R₃)(R_{3'})-CH(X)-CO- ist, R₃ und R_{3'} Wasserstoffatome oder Alk-Reste bedeuten oder zusammen die Gruppen -(CH₂)₄- oder -(CH₂)₅- darstellen, und wenn Z₂ -L²-C(R₈)(R_{8'})-CH(Y)-NH- ist, R₈ und R_{8'} weder beide Wasserstoffatome oder Alkreste sind noch zusammen die Gruppen -(CH₂)₄- oder -(CH₂)₅- bilden; und
mit der Maßgabe, daß M nur dann abwesend ist, wenn Q abwesend ist und wenn Z die folgende Bedeutung hat

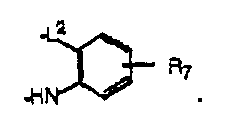

18. Verfahren zur Herstellung einer Verbindung der Formel (I) wie vorstehend in Anspruch 1 definiert, umfassend
a) die oxidative Cyclisierung einer Verbindung der Formel 
S-T1                                    S-T2
|                                       |
Z1-A'-A-(B-Gly-Asp-Q)n-M-Z2

(II)
 in der:
A', A, B, Q, M, Z₁, Z₂ und n wie in Anspruch 1 definiert sind; und
T¹ und T² Wasserstoffatome sind, oder
b) nucleophile Cyclisierung einer Verbindung der Formel (II) durch Behandlung mit einer Base, wobei A', A, B, Q, M, Z₁, Z₂ und n wie vorstehend definiert sind, und einer der Reste T¹ und T² eine ersetzbare Gruppe ist und der andere ein Wasserstoffatom.

19. Verbindung der Formel 
Z1-A'-A-(B-Gly-Asp-Q)nM-Z2
(IA)
 in der
A' abwesend ist oder A^{sn}, Gln, Ala oder Abu bedeutet;
A abwesend ist oder eine D- oder L-Aminosäure bedeutet, ausgewählt aus Arg, HArg, NArg, (Me₂)Arg, (Et₂)Arg, Abu, Ala, Gly, His, Lys oder einem α-R'-substituiertem Derivat davon, Dtc, Tpr oder Pro;
B ein MeArg ist;
Q abwesend ist cder eine D- oder L-Aminosäure bedeutet, ausgewählt aus Tyr, (Alk)Tyr, Phe, (4'W)Phe, HPhe, Phg, Pro, Trp, His, Ser, (Alk)Ser, Thr, (Alk)Thr, (Alk)Cys, (Alk)Pen, Ala, Val, Nva, Met, Leu, Ile, Nle oder Nal oder einem α-R'-substituierten Derivat davon;
M Gly oder D- oder L- Glu, Phe, Pro, Lys, Ser oder, wenn n 1 ist, B-Gly-Glu-Q bedeutet;
W ein Halogenatom oder einen Alk-Rest bedeutet;
R' einen Alk-Rest oder PhCH₂ bedeutet;
n die Bedeutung 1 oder 2 hat, und
Alk einen C₁₋₄-Alkylrest bedeutet;
Z₁ und Z₂ zusammengenommen eine kovalente Bindung zwischen dem aminoterminalen Ende und dem carboxyterminalen Ende bilden;
und pharmazeutisch aktive Salze davon.

20. Verbindung nach Anspruch 19, nämlich
Cyclo(1^{α},6^{γ})-Gly-MeArg-Gly-Asp-Ser-Glu-NH₂ (SEQ ID No. 14);
Cyclo-(1,8)-MeArg-Gly-Asp-Phe-Arg-Gly-Asp-Phe (SEQ ID No. 16).

21. Verfahren zur Herstellung einer Verbindung der Formel 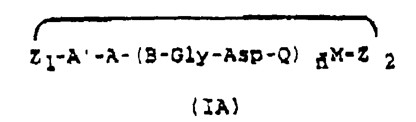
Z1-A'-A-(B-Gly-Asp-Q)nM-Z2
(IA)
 in der:
A', A, B, Q, M und n wie in Anspruch 19 definiert sind;
Z₁ und Z₂ zusammengenommen eine kovalente Bindung zwischen dem aminoterminalen Ende und dem carboxyterminalen Ende bilden, umfassend
i) Cyclisierung einer Verbindung der Formel: H-[A'-A-(B-Gly-Asp-Q-)n-M]-OH in der H- und -OH die Amino- und Carboxylreste beliebiger zweier nebeneinanderliegender Reste im cyclischen Peptid bedeuten und A', A, B, Q, m und n wie in Anspruch 19 definiert sind, mit einem Kupplungsreagenz, wobei reaktive Gruppen gegebenenfalls geschützt sind, und
ii) Entfernung der Schutzgruppen.

## Revendications

1. Composé de formule : 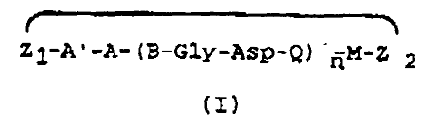
Z1-A'-A-(B-Gly-Asp-Q)n-M-Z2
(I)
 dans laquelle :
A' est absent ou représente un groupe Asn, Gln, Ala ou Abu ;
A est absent ou représente un D- ou L-amino-acide choisi entre Arg, HArg, NArg, (Me₂)Arg, (Et₂)Arg, Abu, Ala, Gly, His, Lys, ou un de leurs dérivés à substituant α-R', Dtc, Tpr ou Pro ;
B représente un D- ou L-amino-acide choisi entre Arg, HArg, (Me₂)Arg, (Et₂)Arg, Lys ou un de leurs dérivés à substituant α-R' ;
Q est absent ou représente un D- ou L-amino-acide choisi entre Tyr, (Alk)Tyr, Phe, (4'W)Phe, HPhe, Phg, Pro, Trp, His, Ser, (Alk)Ser, Thr, (Alk)Thr, (Alk)Cys, (Alk)Pen, Ala, Val, Nva, Met, Leu, Ile, Nle ou Nal, ou un de leurs dérivés à substituant α-R' ;
M est absent ou représente un groupe Gly ou D- ou L-Glu, Phe, Pro, Lys, Ser ou, sous réserve que n est égal à 1, B-Gly-Glu-Q ;
W représente un halogène ou un groupe Alk ;
R' représente un groupe Alk ou PhCH₂ ;
Z¹ représente un groupe 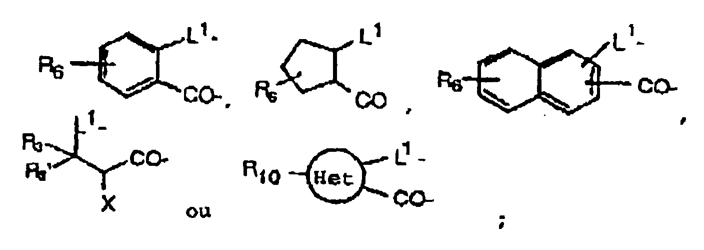
Z₂ représente un groupe 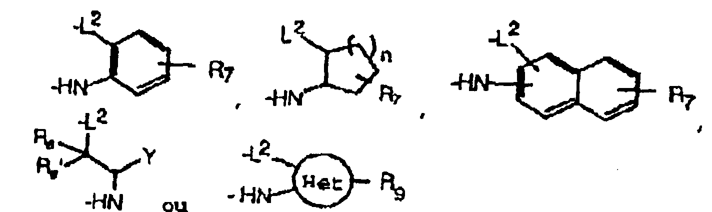
Z₁ et Z₂ étant liés par une liaison covalente entre L¹ et L² ;
L¹ et L² représentent -S- ;
X représente un groupe R₄R₅N ;
Y représente H, un groupe CONR₁R₂ ou CO₂R₂ ;
R₁ et R₂ représentent H, un groupe Alk ou (CH₂)ₚAr ;
R₃ et R_{3'} représentent H, un groupe Alk, (CH₂)ₚAr ou bien, pris conjointement, représentent un groupe -(CH₂)₄- ou -(CH₂)₅- ;
R₄ représente H ou un groupe Alk ;
R₅ représente un groupe R₁₁, R₁₁CO, R₁₁OCO, R₁₁OCH(R_{11'})CO, R₁₁NHCH(R_{11'})CO, R₁₁SCH(R_{11'})CO, R₁₁SO₂ ou R₁₁SO ;
R₆ représente un groupe Alk, OAlk, halogéno ou X ;
R₇ représente H, un groupe Alk, OAlk, halogéno ou Y ;
R₈ et R_{8'} représentent H, un groupe Alk, (CH₂)ₚPh, (CH₂)ₚNph ou, pris conjointement, représentent un groupe -(CH₂)₄- ou -(CH₂)₅- ;
R₉ représente H, un groupe Alk ou Y ;
R₁₀ représente H ou un groupe Alk ;
R₁₁ et R_{11'} représentent H, un groupe alkyle en C₁ à C₅, cycloalkyle en C₃ à C₇, Ar, Ar-(alkyle en C₁ à C₅) ou Ar-(cycloalkyle en C₃ à C₇) ;
Ar représente un groupe phényle ou phényle substitué avec un ou deux groupes alkyle en C₁ à C₅, tri-fluorométhyle, hydroxy, alkoxy en C₁ à C₅ ou halogéno ;
Alk représente un groupe alkyle en C₁ à C₄ ;
Het représente un hétérocycle choisi entre pyridine, pyrrole, pyrrolidine, imidazole, triazole, thiophène, furanne et thiazole, L¹ étant en position ortho par rapport au groupe -CO- et L² étant en position ortho par rapport au groupe -NH- ;
n est égal à 1 ou 2 ; et
p est égal à 0, 1, 2 ou 3,
et ses sels pharmaceutiquement actifs ;
sous réserve que, lorsque Z¹ représente un groupe -L¹-C(R₃)(R_{3'})-CH(X)-CO-, R₃ et R_{3'} représentent des groupes H ou Alk ou, conjointement, représentent un groupe -(CH₂)₄- ou -(CH₂)₅- et Z₂ représente un groupe -L²-C(R₈) (R_{8'})-CH(Y)-NH-, R₈ et R_{8'} ne représentent pas l'un et l'autre H ou un groupe Alk et ne représentent pas non plus, lorsqu'ils sont pris conjointement, un groupe -(CH₂)₄- ou -(CH₂)₅- ; et
sous réserve que M soit absent seulement lorsque Q est absent et seulement lorsque Z² représente un groupe

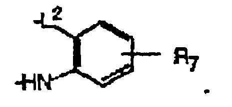

2. Composé suivant la revendication 1, dans lequel Z₁ représente un groupe 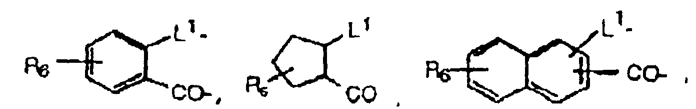 ou

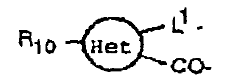

3. Composé suivant la revendication 1, dans lequel Z₂ représente un groupe 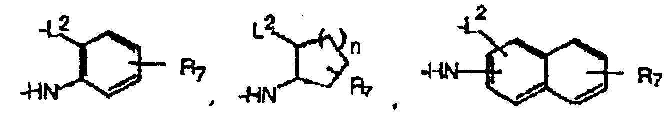 ou

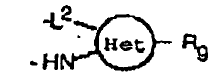

4. Composé suivant la revendication 1, dans lequel Z₁ représente un groupe 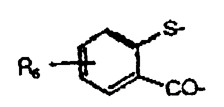 ou Cys et Z₂ représente un groupe 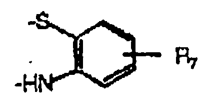 ou Pcs.

5. Composé suivant l'une quelconque des revendications 1 à 4, dans lequel Z₁ représente un groupe

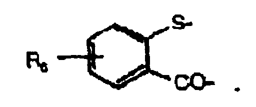

6. Composé suivant l'une quelconque des revendications 1 à 5, dans lequel Z₂ représente un groupe

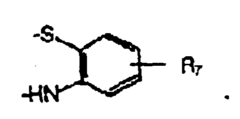

7. Composé suivant l'une quelconque des revendications 1 à 6, dans lequel B représente un groupe Arg, HArg ou un dérivé à substituant α-R' de Arg ou HArg.

8. Composé suivant l'une quelconque des revendications 1 à 7, dans lequel A' et A sont absents.

9. Composé suivant l'une quelconque des revendications 1 à 8, dans lequel B représente un groupe MeArg.

10. Composé, qui consiste en :
cyclo(S,S)-Mba-Arg-Gly-Asp-Cys-NH₂ (SEQ ID NO:1);
N^{α}-Ac-cyclo(S,S)-Cys-Arg-Gly-Asp-Man (SEQ ID NO:2);
cyclo (S,S)-Mba-MeArg-Gly-Asp-Man;
cyclo(S,S)-Mba-MeArg-Gly-Asp-Pcs-NH₂ (SEQ ID NO:3);
cyclo-(S,S)-Mba-Ser-Arg-Gly-Asp-Man (SEQ ID NO:4);
cyclo-(S,S)-Mba-Sar-MeArg-Gly-Asp-Man (SEQ ID NO:5);
cyclo-(S,S)-Mba-Arg-Gly-Asp-Man;
cyclo-(S,S)-Mba-D-MeArg-Gly-Asp-Man;
N^{α}Ac-cyclo-(S,S)-Cys-MeArg-Gly-Asp-(2R,3S)Pcs-NH₂ (SEQ ID NO:6);
N^{α}Ac-cyclo-(S,S)-Cys-MeArg-Gly-Asp-(2R,3R)Pcs-NH₂ (SEQ ID NO:7);
N^{α}Ac-cyclo-(S,S) Cys-Arg-Gly-Asp-Ser-MeArg -Gly-Asp-Ser-Cys-NH₂ (SEQ ID NO:10); ou
N^{α}Ac-cyclo-(S,S)-Cys-MeArg-Gly-Asp-Ser-Arg -Gly-Asp-Ser-Cys-NH₂ (SEQ ID NO:11);

11. Composition pharmaceutique qui comprend un composé suivant l'une quelconque des revendications 1 à 10, 19 ou 20 et un support pharmaceutiquement acceptable.

12. Composé suivant l'une quelconque des revendications 1 à 10, 19 ou 20, destiné à être utilisé dans un médicament.

13. Utilisation d'un composé suivant l'une quelconque des revendications 1 à 10, 19 ou 20 dans la production d'un médicament destiné au traitement d'un infarctus aigu du myocarde.

14. Utilisation d'un composé suivant l'une quelconque des revendications 1 à 10, 19 ou 20 dans la production d'un médicament destiné au traitement d'un ictus ou d'attaques ischémiques transitoires.

15. Utilisation d'un composé suivant l'une quelconque des revendications 1 à 10, 19 ou 20 dans la production d'un médicament destiné au traitement de l'angine de poitrine instable.

16. Utilisation d'un composé suivant l'une quelconque des revendications 1 à 10, 19 ou 20 et d'un agent fibrinolytique dans la production d'un médicament destiné à provoquer une thrombolyse et à inhiber la réocclusion d'une artère ou d'une veine chez un mammifère.

17. Composé de formule : 
S-T^1                                    S-T^2
|                                        |
Z1-A'-A-(B-Gly-Asp-Q) n-M-Z 2

(II)
 dans laquelle :
A' est absent ou représente un groupe Asn, Gln, Ala ou Abu ;
A est absent ou représente un D- ou L-amino-acide choisi entre Arg, HArg, (Me₂)Arg, (Et₂)Arg, Abu, Ala, Gly, His, Lys, ou un de leurs dérivés à substituant α-R', Dtc, Tpr ou Pro ;
B représente un D- ou L-amino-acide choisi entre Arg, HArg, NArg, (Me₂)Arg, (Et₂)Arg, Lys ou un de leurs dérivés à substituant α-R' ;
Q est absent ou représente un D- ou L-amino-acide choisi entre Tyr, (Alk)Tyr, Phe, (4'W)Phe, HPhe, Phg, Pro, Trp, His, Ser, (Alk)Ser, Thr, (Alk)Thr, (Alk)Cys, (Alk)Pen, Ala, Val, Nva, Met, Leu, Ile, Nle ou Nal, ou un de leurs dérivés à substituant α-R' ;
M est absent ou représente un groupe Gly ou D- ou L-Glu, Phe, Pro, Lys, Ser ou, sous réserve que n soit égal à 1, B-Gly-Glu-Q ;
W représente un halogène ou un groupe Alk ;
R' représente un groupe Alk ou PhCH₂ ;
Z¹ représente un groupe 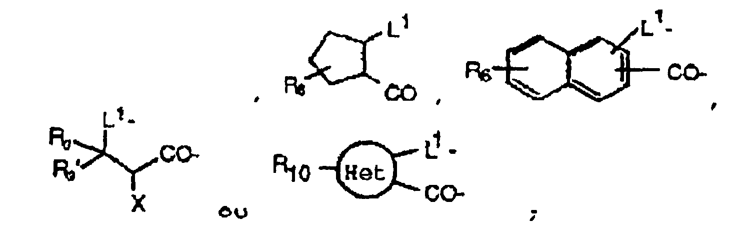
Z₂ représente un groupe 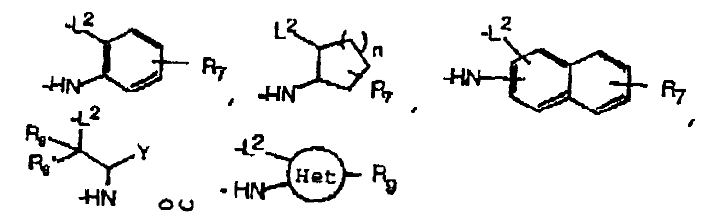 Z₁ et Z₂ étant liés par une liaison covalente entre L¹ et L² ;
L¹ et L² représentent -S- ;
T¹ et T² représentent chacun l'hydrogène ou un groupe déplaçable ;
X représente un groupe R₄R₅N ;
Y représente H, un groupe CONR₁R₂ ou CO₂R₂ ;
R₁ et R₂ représentent H, des groupes Alk ou (CH₂)ₚAr ;
R₃ et R_{3'} représentent H, des groupes Alk,
(CH₂)ₚAr ou, pris conjointement, représentent un groupe -(CH₂)₄- ou -(CH₂)₅- ;
R₄ représente H ou un groupe Alk ;
R₅ représente un groupe R₁₁, R₁₁CO, R₁₁OCO, R₁₁OCH(R_{11'})CO, R₁₁NHCH(R_{11'})CO, R₁₁SCH(R_{11'})CO, R₁₁SO₂ ou R₁₁SO ;
R₆ représente un groupe Alk, OAlk, halogéno ou X ;
R₇ représente H, un groupe Alk, OAlk, halogéno ou Y ;
R₈ et R_{8'} représentent H, des groupes Alk, (CH₂)ₚPh, (CH₂)ₚNph ou, pris conjointement, représentent un groupe -(CH₂)₄- ou (CH₂)₅- ;
R₉ représente H, un groupe Alk ou Y ;
R₁₀ représente H ou un groupe Alk ;
R₁₁ et R_{11'} représentent H, des groupes alkyle en C₁ a C₅, cycloalkyle en C₃ à C₇, Ar, Ar-(alkyle en C₁ à C₅) ou Ar-(cycloalkyle en C₃ à C₇) ;
Ar représente un groupe phényle ou phényle substitué avec un ou deux groupes alkyle en C₁ à C₅, trifluorométhyle, hydroxy, alkoxy en C₁ à C₅ ou halogéno ;
Alk représente un groupe alkyle en C₁ à C₄ ;
Het représente un hétérocycle choisi entre pyridine, pyrrole, pyrrolidine, imidazole, triazole, thiophène, furanne et thiazole, L¹ est en position ortho par rapport au groupe -CO- et L² est en position ortho par rapport au groupe -NH- ;
n est égal à 1 ou 2 ; et
p est égal à 0, 1, 2 ou 3,
sous réserve que, lorsque Z¹ représente un groupe -L¹-C(R₃) (R_{3'})-CH(X)-CO-, R₃ et R_{3'} représentent des groupes H ou Alk ou, conjointement, représentent un groupe -(CH₂)₄- ou -(CH₂)₅- et Z₂ représente un groupe -L²-C(R₈)(R_{8'})-CH(Y)-NH-, R₈ et R_{8'} ne représentent pas l'un et l'autre H ou un groupe Alk et ne représentent pas non plus, lorsqu'ils sont pris conjointement, un groupe -(CH₂)₄- ou -(CH₂)₅- ; et
sous réserve que M soit absent seulement lorsque Q est absent et seulement lorsque Z₂ représente un groupe

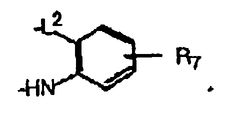

18. Procédé pour la préparation d'un composé de formule (I) répondant à la définition mentionnée précédemment dans la revendication 1, qui comprend
a) la cyclisation oxydative d'un composé de formule : 
S-T1                                    S-T2
|                                       |
Z1-A'-A-(B-Gly-Asp-Q) n-M-Z2
(II)
 dans laquelle :
A', A, B, Q, M, Z₁, Z₂ et n répondent aux définitions suivant la revendication 1 ; et
T¹ et T² représentent H, ou
b) la cyclisation nucléophile d'un composé de formule (II) par traitement avec une base, formule dans laquelle A', A, B, Q, M, Z₁, Z₂ et n répondent aux définitions précitées et un des groupes T¹ et T² est un groupe déplaçable et l'autre représente H.

19. Composé de formule : 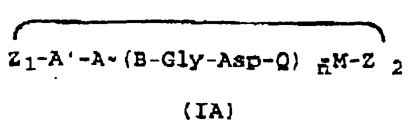
Z 1-A'-A-(B-Gly-Asp-Q) nM-Z 2
(IA)
 dans laquelle :
A' est absent ou représente un groupe Asn, Gln, Ala ou Abu ;
A est absent ou représente un D- ou L-amino-acide choisi entre Arg, HArg, NArg, (Me₂)Arg, (Et₂)Arg, Abu, Ala, Gly, His, Lys, ou un de leurs dérivés à substituant α-R', Dtc, Tpr ou Pro ;
B représente un groupe MeArg ;
Q est absent ou représente un D- ou L-amino-acide choisi entre Tyr, (Alk)Tyr, Phe, (4'W)Phe, HPhe, Phg, Pro, Trp, His, Ser, (Alk)Ser, Thr, (Alk)Thr, (Alk)Cys, (Alk)Pen, Ala, Val, Nva, Met, Leu, Ile, Nle ou Nal, ou un de leurs dérivés a substituant α-R' ;
M représente un groupe Gly ou D- ou L-Glu, Phe, Pro, Lys, Ser ou, sous réserve que n soit égal à 1, B-Gly-Glu-Q ;
W représente un halogène ou un groupe Alk ;
R' représente un groupe Alk ou PhCH₂ ;
n est égal à 1 ou 2 ;
Alk représente un groupe alkyle en C₁ à C₄ ;
Z₁ et Z₂, pris conjointement, représentent une liaison covalente encre le résidu amino-terminal et le résidu carboxy-terminal ;
et ses sels pharmaceutiquement actifs.

20. Composé suivant la revendication 19, qui consiste en
cyclo(1^{α},6^{γ})-Gly-MeArg-Gly-Asp-Ser-Glu-NH₂ (SBQ ID NO:14) ; ou
cyclo-(1,8)-MeArg-Gly-Asp-Phe-Arg-Gly-Asp-Phe (SEQ ID NO:16).

21. Procédé pour la préparation d'un composé de formule : 
Z1-A'-A-(B-Gly-Asp-Q) nM-Z 2
(IA)
 dans laquelle :
A', A, B, Q, M et n répondent aux définitions suivant la revendication 19 ;
Z₁ et Z₂, pris conjointement, représentent une liaison covalente entre le résidu amino-terminal et le résidu carboxy-terminal ;
qui comprend
i) la cyclisation, avec un réactif de couplage, d'un composé de formule (II) H-[A'-A-(B-Gly-Asp-Q-)n-M]-OH dans laquelle H-et -OH représentent le résidu amino et le résidu carboxyle de deux résidus adjacents quelconques dans le peptide cyclique et A', A, B, Q, M et n répondent aux définitions suivant la revendication 19, n'importe quels groupes réactifs étant facultativement protégés, et
ii) l'élimination de tous les groupes protecteurs.
